# EUROPEAN PATENT APPLICATION

(11) **EP 0 617 958 A1**
(43) Date of publication of application: **05.10.1994**
(21) Application number: 92915651.1
(22) Date of filing: 06.07.1992
(51) Int. Cl.: A61K 31/19

(54) **PHARMACEUTICAL COMPOSITION HAVING ANTI-ALCOHOL, RADIOPROTECTING AND ANTI-CHOLERAIC ACTIVITY AND STIMULATING ENERGY METABOLISM, STOMACH MUCOUS MEMBRANE ACID-GENERATION AND SECRETION FUNCTIONS**

(71) Applicant: KOMISSAROVA, Irina Alexeevna, Moscow, 115304 (RU); GUDKOVA, Julia Vasilievna, Moscow 123298 (RU); SOLDATENKOVA, Tatyana Dmitrievna, Moscow, 109028 (RU); BURBENSKAYA, Natalya Mokhailovna, Moscovskaya obl., 140476 (RU); KONDRASHOVA, Tatyana Tikhonovna, Moscow, 129224 (RU); KALANTAR, Irina Lvovna, Moscow, 125414 (RU); TOPOROV, Jury Markelovich, Beshkek, 720020 (KG); SEMENOVA, Galina Fedorovna, Moscow, 113209 (RU); NARTSISSOV, Rjurik Platonovich, Moscow, 115304 (RU); KALININA, Elena Valentinovna, Moscow, 117465 (RU)
(72) Inventor: KOMISSAROVA, Irina Alexeevna, Moscow, 115304 (RU); GUDKOVA, Julia Vasilievna, Moscow 123298 (RU); SOLDATENKOVA, Tatyana Dmitrievna, Moscow, 109028 (RU); BURBENSKAYA, Natalya Mokhailovna, Moscovskaya obl., 140476 (RU); KONDRASHOVA, Tatyana Tikhonovna, Moscow, 129224 (RU); KALANTAR, Irina Lvovna, Moscow, 125414 (RU); TOPOROV, Jury Markelovich, Beshkek, 720020 (KG); SEMENOVA, Galina Fedorovna, Moscow, 113209 (RU); NARTSISSOV, Rjurik Platonovich, Moscow, 115304 (RU); KALININA, Elena Valentinovna, Moscow, 117465 (RU)
(74) Representative: Zimmermann, Hans, Dr.
(86) International application number: PCT/RU92/00134
(87) International publication number: WO 94/01099

(57) **Abstract**

Eine pharmazeutische Komposition mit einer alkoholischen, strahlungsschützenden, anticholerischen, den Energiestoffwechsel, die säurebildende und Sekretionsfunktion der Magenschleimhaut stimulierenden Wirkung enthält als Wirkstoff eine Mischung von Bernstein- und Zitronensäure oder deren pharmazeutisch verträglichen Salzen.

Ein Verfahren zur Prophylaxe und Heilung eines alkoholischen Betrunkenheitszustandes und eines alkoholischen Abstinenzsyndroms, zur Stimulierung des Energiestoffwechsels, zur Stimulierung und Diagnostik der säurebildenden und Sekretionsfunktion der Magenschleimhaut, zum Schutz vor Strahlungsbeschädigungen und zur Prophilaxe von Cholera besteht in peroraler Einführung einer effektiven Menge der anzumeldenden pharmazeutischen Komposition.

## Description

### Technikbereich

Die Erfindung betrifft den Medizinbereich, insbesondere ein neues Arzneipräparat mit einer antialkoholischen, strahlungsschützenden, anticholerischen, den Energiestoffwechsel, die säurebildende und Sekretionsfunktion der Magenschleimdrüse stimulierenden Wirkung.

### Technikstand

Es wird weitestgehend ein antialkoholosches Arzneipräparat verwendet, das Zitronensäure enthält, eine antitoxische Wirkung Wirkung aufweist und während einer hoftigen alkoholischen Betrunkenheit - Alko-Selzer - verwendet wird. Des Präparat kenn jedoch nur episodisch, bei einem üblichen Alkoholverbrauch verwendbarden. Eine chronische Verwendung von Alko-Selzer zwecks Enttoxizierung, welcher Alkoholismuskranke regelmässig bedürfen, führt infolge des in dem Präparat vorhandenen Aspirins zu schwerwiegenden Nachfolgen, nämlich zu dispeptischen Erscheinungen, Änderungen der Magenschleimhaut, zu einer verletzten Funktion des Nervensystems sowie zu Störungen der Hemokougulierungsprozesse. Darüber hinaus begünstigt Alko-Selzer keine Senkung des Alkoholgebrauchs und weist keine alkoschützende Wirkung auf, das heiss, wird zur Prophylaxe von alkoholischer Betrunkenheit nicht verwendet.

Bekannt ist eine antialkoholischen Komposition auf der Basis eines biologischen Materials, die neben der Zitronen- und Bernsteinsaure über 80 verschiedene Naturkomponenten enthält (s. GB 2198041, DE 3641495). Diese Komposition wird als Zusatzstoff bei der Herstellung von Alkohol- und alhoholfreien Getränken und besitzt eine antitoxische Wirkung, sie trägt zu vermindertem Alkoholverbrauch. Trotzdem zeigt sie keine alkoschützenden Eigenschaften auf, ist kostspielig, ihre Herstellung ist durch ihre Rohstoffquelle beschränkt, ihre Zusammensetzung lässt sich erst kompliziert standardisieren.

Bekanntlich ist Alkoholismus von einer Appetitabnahme als einem der wichtigsten Symptome dieser Erkrankung begleitet, und zwar infolge des Rückgang der säurebildenden und Sekretionsfunktion der Magenschleimdrüse und der Astheniezustände.

In der Fachliteratur gibt es Informationen über die Fähigkeit der Bersteinsäure, den Energiestoffwechsel und die Salzsäuresekretion der Magenschleimdrüse zu stimulieren (s. "Terapeutische Wirkung der Bernsteinsäure", AdW der UdSSR, Sammelband, 1976, Pustschino, S. 49-55; 106-107). Die genannten Effekte sind allerdings unstabil und werden von einer Abnahme der funktionalen Aktivität begleitet.

Zur Diagnostik der Säurebildenden und Sekretionsfunktion des Hagens werden solche Präparate wie Hystamin und Pentagastrin weitgehend verwendet. Allerdings ist die Verwendung von Hystamin wegen der oft entstehenden Komplikationen, nämlich Nesselausschlag, Kehlkopfödem, anaphylaktischer Schock, begrenzt. Bei der Injektion von Pentagastrin sind Speichelfluss, Übelkeit, unangenehmes Empfinden in der Bauchfellhöhle, arterieller Druchabfall, ferner eine nachfolgende Abnahme der säurebildenden und Sekretionsfunktion des Magens und Appetitverlust (etwa für 5-6 Tage) zu vermerken.

Zur Choleraprophylaxe verwendet man gegenwärtig allerlei Antibiotika und Vakzinen. Jedoch sind die Antibiotika toxisch, wenig wirkungsvoll, und ihre Verwendung ermöglicht es nicht, die Choleraausbreitung zu begrenzen. Die Vakzinenverwendung fuhrt des öfteren zu allergischen und toxischen Verkomplizierungen, ist begrenzt infolge der langen Zeitperiode für die Formierung der spezifischen Immunität und deren relativ kurzer Wirkungsdauer und erweist sich als wenig effektiv in der Entstehungsperiode des Choloraendemieherdes.

Bekannt ist eine breite Skala von Strahlungsschutzpräparaten, vertreten durch verschiedene Klassen von chemischen Verbindungen. Sie sind allerdings toxisch und werden in grossen Dosen verabreicht. Daher wird in den letzten Jahren grosse Bedeutung der Suche nach Strahlungsschutzpraparaten unter natürlichen Metaboliten beigemessen. Insbesondere wurde eine strahlungsschützende Wirkung der Bernsteinsäure festgestellt, die zu diesem Zweck verwendeten Dosen sind aber sehr hoch (S. International Journal of Radiation Biology, 1987, Nr. 4, vol. 51, Taylor & Francis, Great Britain, pp. 611-617; Radiobiologia, 1990, Nr. 5, vol. 30, Nauka Edition, Moskau, pp. 704-706).

Es sei betont, dass es gegenwärtig in der midizinischen Praxis keine vollständig ungefährliche Präparate gibt, die in der Periode einer Verschlimmerung des Alkoholismus besonders nötig sind, die gleichzeitig eine alkohol-detoxierende, alkoprotektorische, strahlungsschützende und Anticholera-Wirkung aufweisen, welche zu vormindertem Alkoholgebrauch, zur Steigerung des Energiestoffwechsel, zur Erhöhung der säurebildenden und Sekretionsfunktion der Magenschleimdrüse und zur Appetitanregung führen wurde.

### Offenbarung der Erfindung

Die anzumeldende pharmazeutische Komposition ist neuartig und wurde in der Fachliteratur noch nicht beschrieben.

Der Erfindung wurde die Aufgabe zugrunde gelegt, eine neue, hocheffektive, untoxische, keine Nebeneffekte aufzeigende Komposition mit alkohol-detoxirierender, alkoprotektorischer, den Energiestoffwechsel, die säurebildende und Sekretionsfunktion der Magenschleimdrüse anregender sowie strahlungsschützender und Anticholera-Wirkung zu entwickeln.

Die Aufgabe wurde dadurch gelöst, dass die anzumeldende Komposition, enthaltend einen Wirkstoff und ein pharmazeutisches Lösungsmittel, erfindungsgemäss als Wirkstoff eine Mischung der Bernstein- und der Zitronensäure oder deren pharmazeutisch verträglicher Salze enthält.

Diese pharmazeutische Kompostion kann als Pulver, Lösung oder Tabletten verabreicht werden. Bevorzugt wird sie als Lösung oder Pulver peroral, mit einem Wirkstoffanteil von 0,1 bis 0,3 g Bernstein- und von 0,025 bis 0,085 g Zitronensäure oder deren pharmazeutisch verträglichen Salzen fur je 1 Dosis verwendet. Als pharmazeutisches Lösungsmittel enthält die Komposition vorzugsweise Wasser oder alkalisches Mineralwasser.

Die anzumeldende pharmazeutische Kompostion weist eine hohe alkohol-detoxierende alkoprotektorische. den Alkoholgebrauch vermindernde und den Energiestoffwechsel anregende, die säurebildende und Sekretionsfunktion der Magenschleimdrüse stimulierende sowie eine strahlungsschützende und Anticholera-Wirkung auf und findet Verwendung für Prophylaxe und Heilung akuter Alkoholbetrunkenheitszustände und deren Nachfolgen, bei Heilung von astheno-vegetativen Störungen bzw. Verstimmungen sowie für Appetitanregung asthenischen in Perioden des Alkoholabstinenzsyndroms, in der Gastroenterologieklinik als Diagnostikum bei der Ermittlung der Magenschleimaktivität sowie zum Schutz vor Strahlungsbeschädigungen und zur Choleraprophylaxe.

### Kurzbeschreibung der Zeichnungen

Nachstehend wird die Erfindung anhand von Zeichnungen veranschaulicht, die zeigen:
Fig. 1 : Wirkung der anzumeldenden Komposition und von Glutamin auf die Sterblichkeit von Ratten bei einer γ-Bestrahlung;
Fig. 2A: Einwirkung der anzumeldenden Komposition auf tempomassige Spechcharakteristika der Versuchspersonen der Versuchsgruppe 1;
Fig. 2B: Einwirkung der anzumeldenden Komposition auf tempomässige Sprechcharakteristika der Versuchspersonen der Vorsuchsgruppe 2;
Fig. 2C: Einwirkung der anzumeldenden Komposition auf tempomässige Sprechcharakteristika der Versuchspersonen der Versuchsgruppe 3.

### Bevorzugte Variante der Erfindungsverwirklichung

Die anzumeldende Komposition ist eine Mischung von Bernstein- und Zitronensäuren. Diese Carbonsäuren sind natürliche Metabolite in pflanzlichen und tierischen Geweben, sie sind Pulversubstanzen, weissfarben, geruchlos, gut wasserlöslich, in Alakalilösungen und schwerlöslich in Äthanol und in Ölen.

Die anzumeldende Komposition wurde experimentell an Tieren sowie klinisch an Menschen untersucht.

Die antialkoholische Einwirkung der Kompostion wurde an männlichen Amphibien mit Körpermasse 30-40 g, an Mäusen mit Körpermasse 20-25 g und an männlichen Ratten mit Körpermasse 200-250 g untersucht.

An einem Standardmodell (freigelegte Hemisphären und Riechnerven) eines Frosches Rana temporaria wurde der Riechnerv mit einer Häufigkeit von einmal pro 2 Minuten und unter Registrierung des erzeugten Potentials des primordialen Hypokampes 40-60 Minuten lang stimuliert. Es wurde die Amplitude von fünf Grundelementen des erzeugten Potentials registriert, und zwar:
die positive Komponente PK₁. die das Kommen der efferenten Salve und das Entstehen der afferenten stimulierenden postsynaptischen Potentiale widerspiegelt;
die negative Komponente NK, die mit der Entwicklung der früheren postsynaptischen Bremspotentiale zusammenhängt;
das positive Potential PK₂, das die Entwicklung von erregenden postsynaptischen Rückpotentiale und Salvenentladungen in den Neuronen des primordialen Hypokampes: widerspiegelt;
das negative Potential NP₂, das spätere postsynaptische Bremspotentiale kennzeichnet; und
das spätere positive Potential PK_{sp.}, das das dauernde Depolarisationspotential der Neuronen widerspiegelt.

Vor der Registrierung des erzeugten Potentials wurde die anzumeldende Komposition an eine erste Gruppe von 10 Tieren intramuskulär in einer Dosis von 3,5 mg/kg Körpergewicht 20 Minuten vor einer Impfung von 10%igem Äthanol in einer Dosis von 0,5 g/kg Körpergewicht, an ein zweit Gruppe von 10 Tieren in gleicher Dosis 20 Minuten vor einer Impfung von 10%igem Äthanol in gleicher Dosis ein entsprechendes Volumen der physiologischer Lösung, während an eine Kontrollgruppe von 10 Tieren nur ein entsprechendes Volumen der physiologischen Lösung geimpft. Die Untersuchungsergebnisse sind in Tabelle 1 angegeben.

**Tabelle 1**

| Einfluss der anzumeldenden Komposition auf die Amplitude der Komponenten des erzeugten Potentials des primordialen Hypokampes bei Fröschen, in % von Kontrollwerten, M∓m | | |
|---|---|---|
| Komponenten des erzeugten Potentials | Amplitude der Komponenten des erzeugten Potentials | |
| | Gruppe 1 | Gruppe 2 |
| | Die anzumeldende Komposition + Äthanol | Physiolog. Lösung + Äthanol |
| 1 | 2 | 3 |
| PK₁ | 92,35± 0,98* | 88,72∓0,17 |
| NK | 92,86∓ 7,15 | 86,86∓ 12,09 |
| PK₂ | 94,04∓ 0,71* | 49,87∓ 1,48 |
| NP₂ | 87,37∓ 11,18* | 79,53∓ 0,22 |
| PK_{sp.} | 78,79∓12,12* | 83,22∓1,40 |
| Anmerkung: Die vollständigen Bezeichnungen der Komponenten des erzeugten Potentials sind im Text angegeben; | | |

| | | |
|---|---|---|
| * der Unterschied zur Gruppe 2 ist unbestreitbar, p < 0,05. | | |

Wie aus Tabelle 1 ersichtlich, ergibt Äthanol bei einer Vorimpfung der anzumeldenden Komposition keine sichtliche Änderungen der Amplitude der Komponenten des erzeugten Potentials, was von deren alkoprotektorischer detoxierenden Wirkung zeugt.

Eine Einwirkung der anzumeldenden Kompostion auf die Verlaufweise eines akuten alkoholoschen Betrunkenheitszustands bei Mäusen und Ratten wurde untersucht. Den Versuchstieren wurde die Komposition in einer Dosis von 3,75 mg/kg Körpergewicht 20 Minuten vor einer intraabdominalen Impfung von Äthanol in Dosen von 2; 3; 4, 5 und 6 g/kg Körpermasse intragastral eingeführt, während den Kontrolltieren ein entsprechendes Volumen Wasser 20 Minuten vor einer Äthanoleinführung verabreicht wurde. Dabei wurde die Zeitperiode der Seitenlage der Tiere berücksichtigt. Die ermittelten Werte stehen in Tabelle 2.

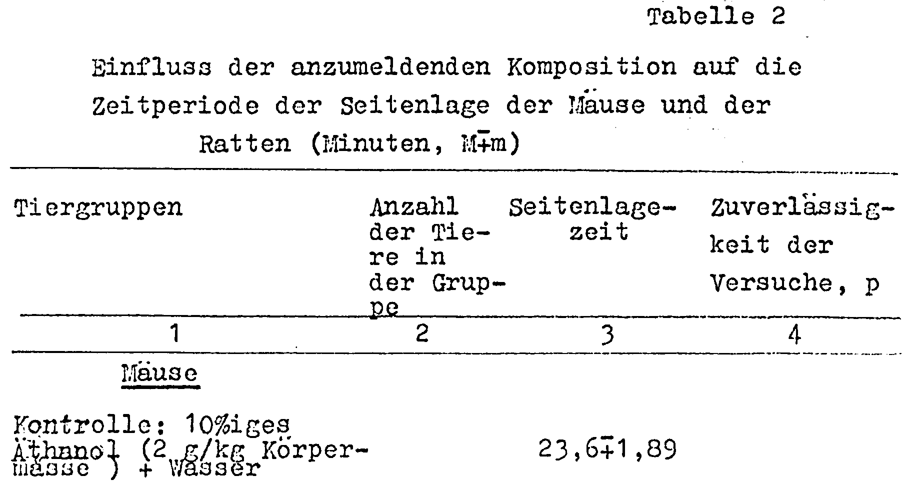

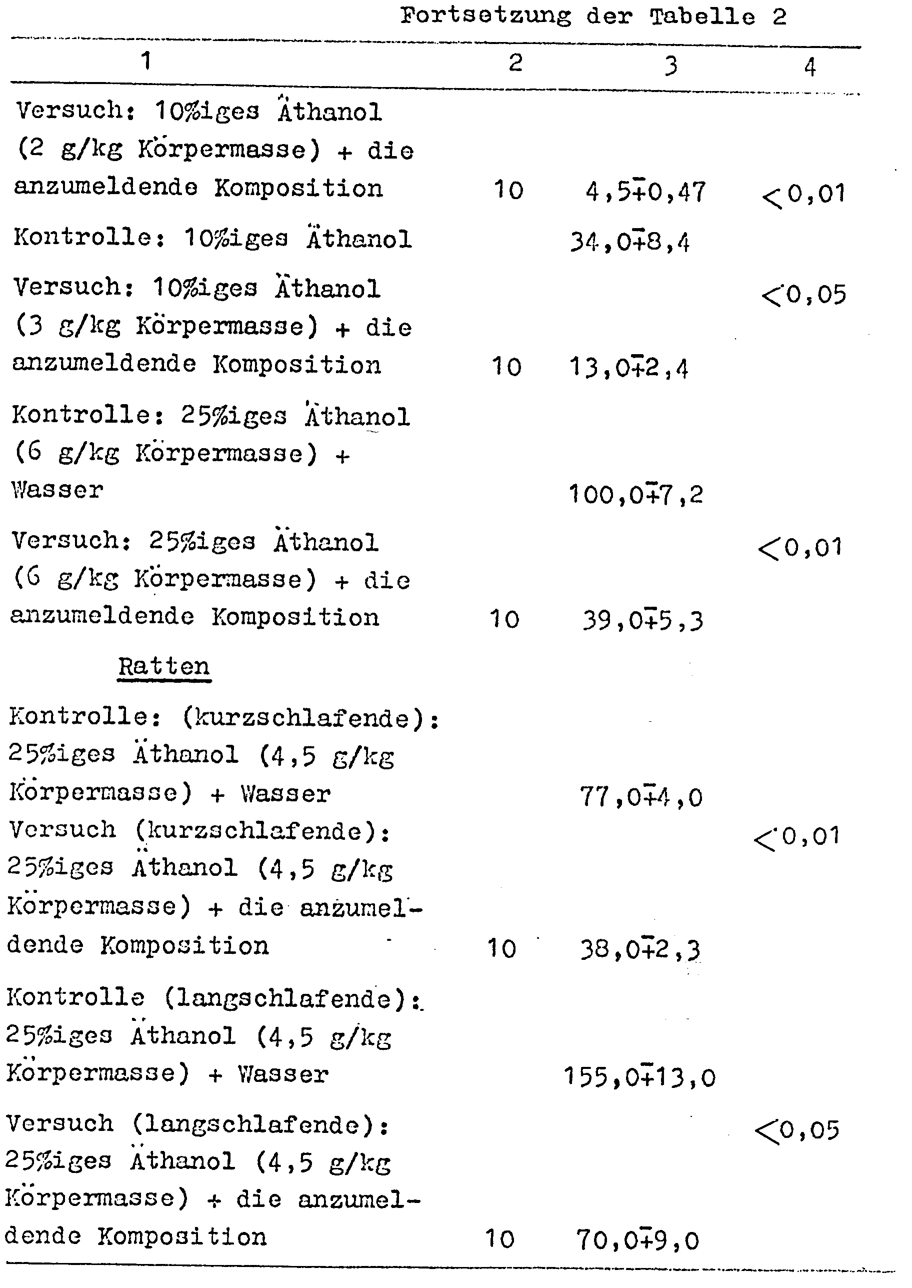

Die Angaben der Tabelle 2 zeigen, dass die Voreinführung der anzumeldenden Komposition die Seitenlagezeit der Tiere 2- bis 4-malig reduziert und alkoprotektorisch und detoxierend wirkt, dabei hängt diese Wirkung von der Empfindlichkeit der Versuchstiere (kurz- und langschlafende Ratten) für Äthanol nicht.

Eine Einwirkung der anzumeldenden Komposition auf die Art der Verhaltensreaktionen bei Mäusen im Zustand einer akuten Alkoholbetrunkenheit wurde untersucht. Die anzumeldende Komposition wurde dabei einer ersten Gruppe von 40 Tieren intragastral eingeführt, und zwar in einer Dosis von 1,5 mg/kg Körpermasse 20 Minuten vor einer intraabdominalen Einführung von 10%igem Äthanol in einer Dosis von 0,5 g/kg Körpermasse. Einer zweiten Versuchsgruppe aus 40 Tieren wurde 20 Minuten vor einer intraabdominalen Einführung von 10%igem Äthanol ein entsprechendes Volumen der physiologischen Lösung in derselben Dosis intragastral eingeführt, während man der Kontrollgruppe von 40 Tieren lediglich ein entsprechendes Volumen der physiologischen Lösung intragastral einführte. Das Verhalten der Mäuse untersuchte man durch einen Test des "offenen Feldes". Die Versuchsergebnisse sind in Tabelle 3 angegeben:

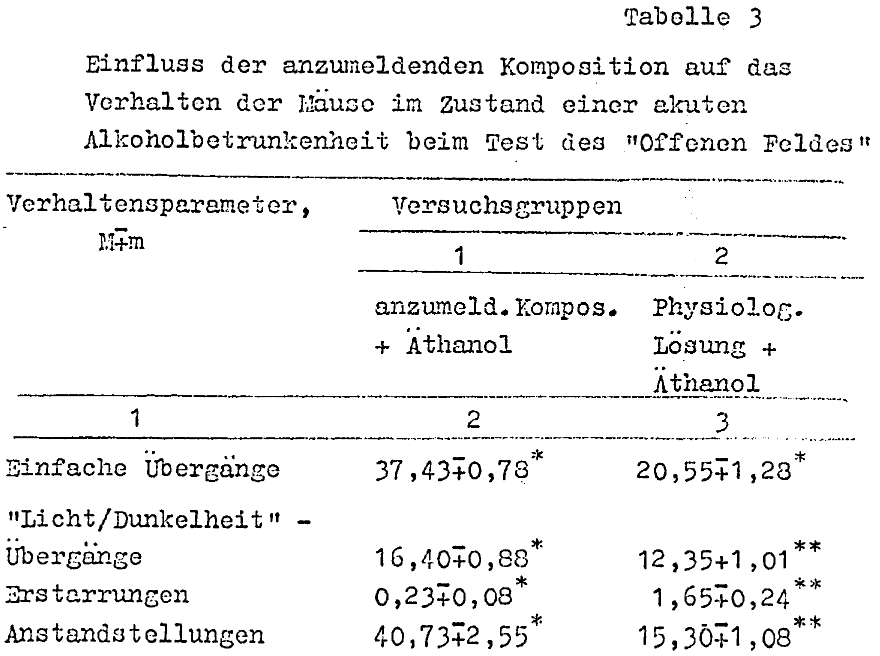

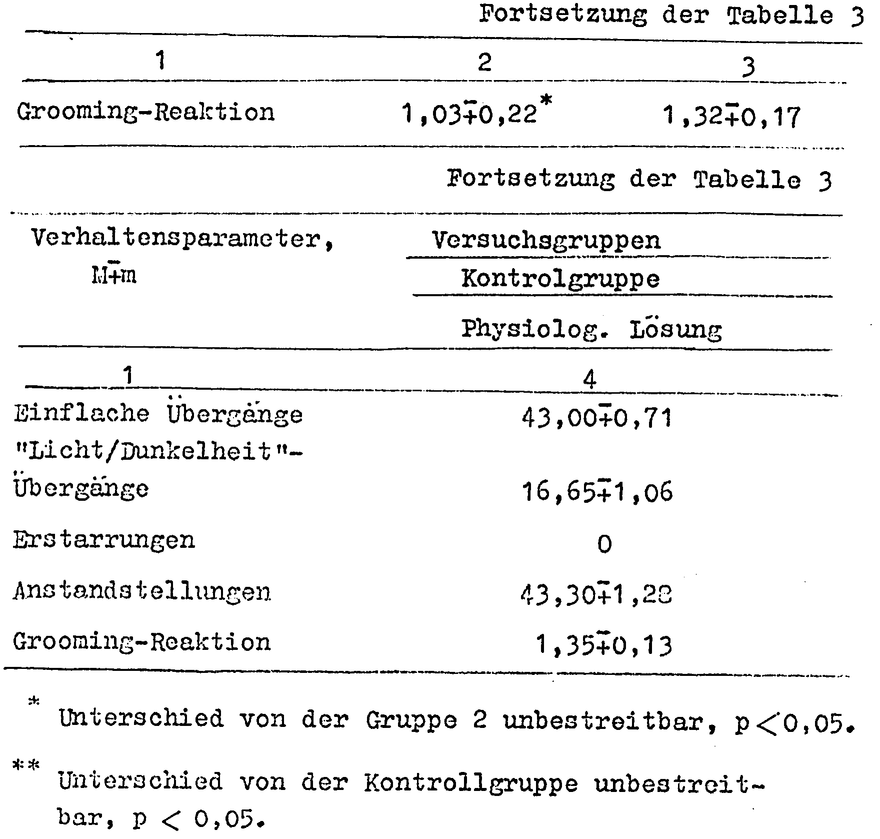

Wie aus Tabelle 3 ersichtlich, zeigt die voreingeführt anzumeldende Komposition eine alkoprotektorische detoxierende Wirkung auf und vermindert oder besetigt, vollständig die durch die Äthanoleinwirkung ergebenen Änderungen des Verhaltens der Tiere.

Eine Vergleichsuntersuchung der Einwirkung der anzumeldenden Kompostion und deren einer Komponente, nämlich der Bernsteinsäure, auf den Verlauf einer akuten Alkoholintoxikation bei Ratten wurde vorgenommen. Eine erste Versuchsgruppe bekam die anzumeldende Komposition intragastral, in einer Dosis von 1,85 mg/kg Körpermasse, einer zweiten Gruppe führte man intragastral Bernsteinsäure in gleicher Dosis ein, eine Kontrollgruppe bekam intragastral ein entsprechendes Volumen Wasser. Nach 40 Minuten bekamen alle drei Gruppen einmalig intraabdominal 25%iges Äthanol in einer Dosis von 3,5 g/kg Körpermasse, dabei wurde die Seitenlagezeit der Tiere in Betracht gezogen. Nach 60 Minuten nach der Erholeng der Tiere aus der Seitenlage wurde ihnen erneut 25%iges Äthanol in einer Dosis von 3 g/kg Körpermasse eingeführt und ihre Seitenlagezeit wiederholt festgestellt. Die Untersuchungsergebnisse sind in Tabelle 4 angegeben:

**Tabelle 4**

| Einfluss der anzumeldenden Komposition und der Bersteinsäure auf die Seitenlagezeit bei Ratten bei einer einmaligen und einer wiederholten Verabreichung von Athanol | | | |
|---|---|---|---|
| Versuchstiergruppen | Anzahl der Tiere in der Gruppe | Seitenlagezeit, Min., M∓m | |
| | | Äthanol einmalig eingeführt | Wiederholtes Einführen von Äthanol |
| Kontrolle (Wasser) | 10 | 45,6∓7,1 | 93,0∓15,5 |
| Gruppe 1 (anzumeldende Komposition) | 10 | 8,2∓2,7 | 11,0∓4,1 |
| Gruppe 2 (Bernsteinsäure) | 10 | 1,6∓1,1 | 24,9∓3,8 |
| Versuchszuverlässigkeit, p | | <0,01 | <0,01 |

Tabelle 4 zeigt, dass die alkoprotektorische detoxierende Wirkung der Bernsteinsäure bei einmaliger Einführung von Äthanol mehr zum Ausdruck kommt als bei der anzumeldenden Komposition, jedoch ist die Wirkung der letzteren dauernder und deren Vorteil im Vergleich zu der Bernsteinsäure zeigt sich bei einer erneuter Äthanoleinführung.

Ferner wurde ein Einfluss der anzumeldenden Komposition auf den Alkoholgebrauch bei Ratten als "chronischen Alkoholikern" untersucht. Bei dem Experiment wurden Tiere eingesetzt, welche sich lange Zeit in Verhältnissen einer frieen Wahl zwischen einem 15%igen Äthanol und Wasser befanden und Alkohol in einer Dosis von 7 bis 10 g/kg Körpermasse alle 24 Stunden konsumierten. Einer Versuchsgruppe von Tieren wurde die anzumeldende Komposition zwei Wochen lang zweimal pro Tag in einer Dosis von 3,75 mg/kg Körpermasse intragastral eingeführt, während einer Kontrollgruppe zu gleichen Fristen ein entsprechendes Wasservolumen intragastral eingeführt wurde. Bei einer freien Wahl zwischen 15%igem Äthanol und Wasser wurde der individueller Tagesverbrauch der Tiere von Äthanol innerhalb von 10 Tagen vor der Einführung der anzumeldenden Komposition, dann während der ersten und der zweiten Woche der Einführung der Komposition sowie während der ersten Woche nach deren Einstellung berücksichtigt. Die Untersuchungsergebnisse stehen in Tabelle 5. Sie zeigen, dass bei der Einführung der anzumeldenden Komposition kein Anstieg des Alkoholgebrauchs zu verzeichnen ist.

**Tabelle 5**

| Einfluss der anzumeldenden Komposition auf den Tagesgebrauch des 15%igen Äthanol bei Ratten (ml, M∓m) | | | | | |
|---|---|---|---|---|---|
| Tiergruppen | Anzahl der Tiere in der Gruppe | Untersuchungsetappen | | | |
| | | 10 Tage vor Einführung der Komposition | 1.Woche der Einführung der Komposition | 2.Woche der Einführung der Komposition | 1 Woche nach der Einstellung der Einführung der Komposition |
| Kontrolle | 10 | 17,7∓ | 18,0∓ | 17,2∓ | 18,2∓ |
| | | 0,35 | 0,12 | 0,41 | 0,20 |
| Versuch | 10 | 16,9∓ | 19,2∓ | 18,0∓ | 15,5∓ |
| | | 0,48 | 0,47 | 0,42 | 0,65 |

Untersucht wurde ferner ein Einfluss der anzumeldenden Komposition auf die bioenergetischen Prozesse in der Leber der Versuchstiere. Die Untersuchungen wurden an Homogenaten der Leber von Mausmännchen vorgenommen. Einer Versuchsgruppe von 20 Tieren wurde die anzumeldende Komposition intragastral 60 Minuten vor der Dekapitation in einer Dosis von 3,75 mg/kg Körpermasse eingeführt, während eine Kontrollgruppe von 20 Tieren ein entsprechendes Wasservolumen intragastral bekam. Die Atemintensitat der Homogenate der Mausleber wurde polarografisch, und zwar durch Messung des von ihnen absorbierten Sauerstoffs unter Verwendung der Clarc-Elektrode, ermittelt. Eine Einführung der anzumeldenden Komposition bei den Versuchstieren führt zu einer sichtlichen Steigerung der Geschwindigkeit des endogenen Atems (Atem auf endogenen Substraten), diese kommt in einem rapiden Ansteig des Anfangssauerverbrauchs durch die Homogenate der Mausleber (68,4∓13,1 Sauerstoff- nanog-Atom/mg Eiweiss) Sek. gegenüber 32,6∓10,7 in der Kontrollgruppe, p <0,07) zum Ausdruck.

Die succinatabhängige Konstante des endogenen Atem wurde durch Untersuchung der Inhibierung des Atems der Homogenate dar Versuchs- und Kontrolltiere bei verschiedenen Malonatkonzentrtaionen als eines spezifischen Inhibitors der Succinatdehydrogenase in einem Inkubationsmedium ermittelt. Die erhaltenen Werte sind in Tabelle 6 angeführt. Wie diese zeigt, sind es bei den Kontrolltieren nur 35 bis 46% des andogenen Atems der Leberhomogenate, die durch Oxydierung der endogenen Bernsteinsäure gewährleistet werden, während die succinatabhängige Konstante bzw. Fraktion des endogenen Atems bei den Versuchstieren rund 80% (p <0,001) beträgt. Kennzeichnend ist, dass im Verlaufe von 55-65 Minuten nach der Dekapitation der Tiere eine allmähliche Erhöhung des Anteils der succinatabhängigen Fraktion an dem gesamten endogenen Atem der Leberhomogenate bei den Versuchstieren erfolgt.

Darüber hinaus konnten experimentelle Untersuchungen aufzeigen, dass bei einer Einführung der anzumeldenden Komposition der endogene Atem der Homogenate der Mausleber (zum Unterschied von den Kontrolltieren und von einer Einführung lediglich der Bernsteinsäure) zeigt eine Empfindlichkeit für die Einwirkung nicht nur des Malonates, sondern auch das Amitals (eines allbekannten NAD-Inhibitors) der abhängigen Substrate zeigt.

Es wurde eine Vergleichsuntersuchung der Wirkung dar anzumeldenden Komposition und der Bernsteinsäure auf die Aktivität der Succinutdehydrogenase in den Magengeweben, in der Leber und in den Blutlymphozyten der weiblichen Mäuse mit einer Körpermasse von 20-30 g durchgeführt. Man führte einer ersten Gruppe von Tieren die anzumeldende Komposition in einer Dosis von 4 mg/kg Körpermasse intragastral ein, wahrend eine zweite Gruppe von Tieren Bernsteinsäure in gleicher Dosis intragastral und eine Kontrollgruppe ein entsprechendes Volumen von destlliertem Wasser intragastral bekamen. 40 Minuten nach der Einführung der genannten Präparate wurden die Tiere dekaptiert, und die Aktivität der Succinatdehydrogenase wurde gemessen, und zwar hystochemisch an kryostaten schnittpräparaten von Leber und Magen (s. Archiv für Anatomie, Hystologie und Embryologie, Nr. 12, 1969, Verlag Medizin, Lemingrad, S. 112-116) und zytochemisch an Lymphozyten (s. Archiv fur Anatomie, Hystologie und Embryologie, Nr. 5, 1969, Verlag Medizin, Leningrad, S. 85-91). Die erhaltenen Werte stehen in Tabelle 7. Diese zeigt, dass die anzumeldende Komposition die Succinatdehydrogenase in der Magenschleimhaut, Leber und in den Blutlymphozyten aktiviert und einen eindeutigen Vorteil gegenüber der Bernsteinsäure besitzt.

Eine Analyse der durchgeführtan Untersuchungen hat gezeigt, dass die anzumeldende Komposition die bioenergetischen Prozesse in den Mitochondrien verschiedener Gewebe stimuliert. Ihre Wirkung wird uber eine Erhöhung das Anteils der endogenen Substrate des Zyklus der Tricarnobsäuren, über einen Anstieg deren Oxydierungsgeschwindigkeit sowie über eine erhöhte Aktivität der Succinnatdehydrogenase verwirklicht.

**Tabelle 6**

| Inhibierung des endogenen Atems der Leberhomogenate bei Mäusen durch unterschiedliche Malonatkonzentrationen (%, M∓m) | | | |
|---|---|---|---|
| Malonatkonzentration im Inkebationsmedium, M | Untersuchungsetappen | | |
| | 20 Minuten nach dar Dekapitation der Tiere | | |
| | Kontrolle | Versuch | Zuverlässigkeit der Experimente, p |
| 1 | 2 | 3 | 4 |
| 5 x 10⁻⁴ | 8,5∓2,2 | 27,2∓2,9 | <0,05 |
| 1 x 10⁻³ | 9,6∓3,7 | 51,5∓3,8 | <0,05 |
| 1,5 x 10⁻³ | 18,5∓5,7 | 65,7∓3,1 | <0,05 |
| 2 x 10⁻³ | 36,2∓10,3 | 73,5∓4,1 | <0,05 |

| | 55-65 Minuten nach dar Dekapitation der Tiere | | |
|---|---|---|---|
| 5 x 10⁻⁴ | 17,0∓4,6 | 37,0∓5,8 | <0,05 |
| 1 x 10⁻³ | 20,0∓5,1 | 59,5∓8,0 | <0,05 |
| 1,5 x 10⁻³ | 38,7∓8,1 | 67,5∓8,7 | <0,05 |
| 2 x 10⁻³ | 75,5∓11,4 | | <0,05 |

**Tabelle 7**

| Einfluss der anzumeldenden Komposition und der Bernsteinsäure auf die Leber- und Magen-Succinatdehydrogenase (in nano-Mol von Formazan von p-nitroviolettem Tetrazolium/Min./mg Eiweiss, M∓m) und der Blutlymphozyten (in Granulen von Formazan von p-nitroviolettem Tetrazolium/St./50 Zellen, M∓m) bei Mäusen | | | | |
|---|---|---|---|---|
| Gruppen von Tieren | Anzahl von Tieren in der Gruppe | Gewebe | | |
| | | Leber | Magen | Blutlymphozyten |
| 1 | 2 | 3 | 4 | 5 |
| Kontrolle | 20 | 20,3∓2,1 | 14,3∓1,7 | 546∓3,4 |
| Gruppe 1 (die anzumeldende Komp.) | 20 | 26,6∓1,2 | 30,8∓1,0 | 656∓6,7 |
| Gruppe 2 (Bernsteinsäure) | 20 | 21,8∓0,6 | 26,3∓3,0 | 643∓5,1 |
| Zuverlässigkeit der Experimente, p | | <0,005 | <0,05 | <0,05 |

Eine strahlungsschützende Wirkung der anzumeldenden Komposition an männlichen Ratten mit einer Körpermasse 180-200 g wurde untersucht. Die Tiere wurden einer - Bestrahlung in einer Dosis von 16 Gr an einer Anlage LUE "Malva-2" bei einer Dosisstärke 1,4 Gr/Min., Feld 15x20, Brennweite 30 cm, Spannung 8 MeV und Stromstärke 3,5 µA ausgesetzt.

Einer Versuchsgruppe von 10 Tieren wurde die anzumeldende Komposition 30 Minuten vor der Bestrahlung per os in einer Dosis von 2 mg/kg Körpermasse eingeführt, eine Kontrollgruppe aus 10 Tieren bekam analogischerweise ein entsprechendes Volumen destilliertes Wasser (0,5 ml). Es wurde festgestellt, dass bei der Dosis 16 Gr, Lethaldosis für 100% der Kontrolltiere, die Einführung der enzumeldenden Komposition ein 33%iges Überleben der Tiere ergibt und eine durchschnittliche Lebensdaur von bis 9 Tage gegenüber 6,9 Tage unter den Kontrolltieren verlängert.

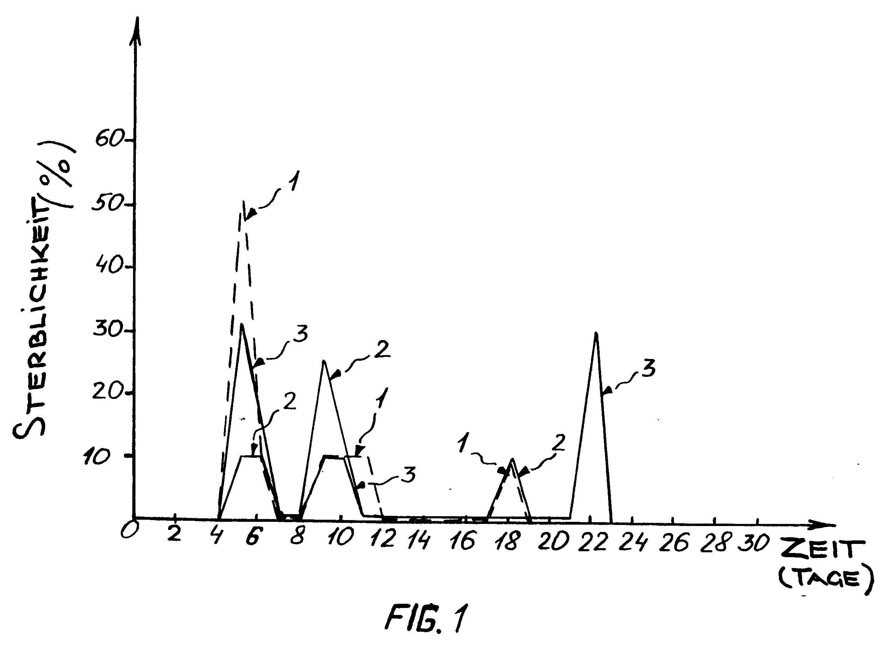

Die in Fig. 1 angeführten Rattensterblichkeitskurven veranschaulichen, dass das Todesmaximum der Kontrolltiere auf die erste Sterblichkeitsspitze kommt (Fig. 1, Kurve 1), was für hohe Dosen kennzeichnend ist, während die protektorische Wirkung der anzumeldenden Komposition dieses Maximum nicht nur senkt, sondern auch auf die zweite Sterblichkeitsspitze verschiebt (Fig. 1, Kurve 2).

Als Vergleichspräparat wurde in dem Experiment der bekannte Strahlungsprotektor Glutation (GSH) verwendet (Studia Biophysica, 1975, Nr. 1, vol. 53, Academy Press, Berlin, DDR, p.p. 121-124). Das Präparat wurde einer Gruppe von 10 Tieren i.p. 30 Minuten vor Bestrahlungsbeginn in einer Dosis von 100 mg/kg Körpermasse eingeführt. Bei der Dosis von 16 Gr und den obengenannten Bestrahlungsbedingungen führte die Glutationeinführung zum 100%igen Tod der Tiere, die Lebensdauer der Ratten betrug jedoch 10, 2 Tage gegenüber 6,9 Tage in der Kontrollgruppe.

Glutation ergibt einen Rückgang der Rattensterblichkeit in der ersten Kurvenspitze, jedoch in viel vermindertem Masse als die anzumeldende Komposition und eine Sterblichkeitserhöhung in dar dritten Spitze (Fig. 1, Kurve 3). Das zeugt von seiner im Vergleich zu der anzumeldenden Komposition schwächeren strahlungsprotektorischen Wirkung.

Die erhaltenen Untersuchungswerte ermöglichen es, die anzumeldende Komposition als einen Strahlungsprotektor mittlerer Effektivität einzuschätzen. Dabei ist die äusserst kleine Dosis dieses Präparates von besonderem Interesse (eine effektive Dosis dar Bernsteinsäure für Nagetiere beträgt 2,7 g/kg Körpermasse), die eine bestimmte Korrektion der Wege des Energiestoffwechsels in Richtung zur Erhöhung der Strahlungsresistenz des Organismus bedingt.

Es wurde eine Einwirkung der anzumeldenden Komposition auf die säurebildende und Sekretionsfunktion der Magenschleimhaut bei Hunden und Mäusen untersucht. Zu dem Experiment wurden Hunde mit einer Körpermasse von 10-20 kg mit einer Magenfistel nach Below und Mäuse mit Körpermasse 20-30 g eingesetzt.

Untersucht wurde der Einfluss verschiedener Dosen der anzumeldenden Komposition auf die säurebildende und Sekretinsfunktion der Magendrüsen bei Hunden. Die Komposition wurde in die Magenfistel nüchtern in 5 ml destilliertem Wasser eingeführt, und nach 30 Minuten wurden die Werte der Magensekretion gemessen. Die Untersuchungsergebnisse sind in Tabelle 8 angeführt.

**Tabelle 8**

| Einfluss der Dosisgrössen der Komposition auf die saurebildende und Sekretionsaktivitat der Magenschleimdrüse bei Hunden (eine Gruppe von 10 Tieren) | | | | |
|---|---|---|---|---|
| Dosen der Komposi-tion, mg/kg Körpermasse | | Säurebildende Aktivität des Magens, M∓m | Sekretionsaktivität des Magens, M∓m | |
| | | pH der Magenflüssigkeit | Sekretion der Magenflüssigkeit, ml/Stunde | Pepsinsekretion, mg/Stunde |
| (nüchtern) | 0 | | | |
| | | 7,3∓0,4 | 7,0∓1,0 | 0,48∓0,06 |
| | 0,36 | 5,6∓0,6 | 15,6∓1,3 | 1,28∓0,07 |
| Zuverlässigkeit der Experimente, p | | <0,02 | <0,001 | <0,01 |
| | 3,6 | 4,7∓1,1 | 20,9∓4,1 | 2,28∓0,08 |
| Zuverlässigkeit der Experimente, p | | <0,05 | <0,01 | <0,001 |
| | 5,4 | 4,75∓1,5 | 21,2∓3,1 | 2,31∓0,07 |
| Zuverlässigkeit der Experimente,p | | - | - | - |
| Anmerkung: p-Wert ist im Vergleich mit der vorherigen Dosis angegeben. | | | | |

Wie Tabelle 8 zeigt, ist die Dosis 3,6 mg/kg Körpermasse für die enzumeldende Komposition optimal für die Stimulierung der funktionalen Aktivität der Magendrüsen. Ausserdem wurde gefunden, dass die Wirkung von 0,1 der optimalen Dosis der anzumeldenden Komposition nach 30 bis 40 Minuten nach deren Einführung endet, während nach 80 Minuten nach der Einführung der optimalen Dosis die Menge der Magenflüssigkeit und deren HCl-Gehalt und Pepsin- immer noch höher sind als solche im Nüchternzustand. Es sei betont, dass eine fortlaufende Einführung von 0,1 der optimalen Dosis der anzumeldenden Komposition in die Magenfistel der Hunde auch nach 40 Minuten (Sekretionsabfall) zu keiner Verminderung der optimalen Dosis führt, sondern, im Gegenteil, den stimulierenden Effekt der anzumeldenden Komposition potenziert und dadurch die Magendrüsenaktivität viel bedeutender und für viel längere Zeit erhöht.

Untersucht wurde eine Einwirkung der anzumeldenden Komposition auf die säurebildende und Sekretionsactivtät der Magendrüsen und auf die Aktivität der Succinatdehydrogenase der Magenschleimhaut und der Lymphozyten des peripherischen Blutes bei Hunden. Die anzumeldende Komposition wurde in die Magenfistel der Hunde nüchtern in einer Dosis von 3,6 mg/kg Körpermasse in 5 ml destilliertem Wasser eingeführt. Nach 40 Minuten wurden die Magensekretionswerte und die Aktivität der Succinatdehydrogenase gemessen. Die Ergebnisse stehen in Tabelle 9. Sie zeigen, dass die anzumeldende Komposition sowohl die funktionale Aktivität der Magendrüsen als auch die Aktivität der Succinatdehydrogenase der Magenschleimhaut wesentlich erhöht. Sie beeinflusst folglich die Magen funktion durch Induzieren von bioenergetischen Prozessen, und zwar der succinatabhängigen Oxydierungswege in den Mitochondrien verschiedener Gewebe.

Eine Vergleichsuntersuchung der Einwirkung der anzumeldenden Komposition in der Dosis 3,6 mg/kg Körpermasse sowie der Bernsteinsäure in gleicher Dosis auf die Aktivität der Magenschleimhaut und auf die Aktivität der Succinatdehydrogenase darin bei Hunden (Gruppe von 10 Tieren) wurde durchgeführt. Es wurde nachgewiesen, dass eine Bernsteinsäurestimulierung der Magendrüsenfunktion und der fermentation Aktivität weniger feststellbar ist und dass deren Einwirkung auf die Magensekretionswerte, im Unterschied von der anzumeldenden Komposition, 20 Minuten nach der Einführung endet.

Untersucht wurde ein Einfluss der anzumeldenden Komposition und der Bernsteinsaure auf die säurebildende und Sekretionsaktivität der Magenschleimhaut sowie auf den Gehalt von Makromolekülen in Magen- und Lebergeweben bei Mäusen. Eine erste Tiergruppe bekam nüchtern intragastral (Sonde) die Dosis 3,6 mg/kg Körpermasse der Komposition in einem Volumen von 0,2 ml, einer zweiten Tiergruppe wurde Bernsteinsäure analogischerweise, in gleicher Dosis, während einer Kontrollgruppe ein entsprechendes Volumen Wasser eingeführt werde. Die Mäuse wurden 35-45 Minuten nach der Einführung der Präparate dekapitiert, ihre Magen und Lebern wurden freigelegt. Der Magen wurde durch die kardiale Öffnung mit destilliertem Wasser gewaschen, die Abwaschflüssigkeit wurde aus der Antralöffnung gesammelt. Der Mageninhalt (Abwaschflüssigkeit, 5 ml) wurde zur Ermittlung von pH, Pepsingehalt und Gesamteiweissstoff verwendet. Dann wurde der Magen wieder mit Wasser gefüllt, in einem Kryostat eingefroren, worauf Serienschnittpräparate 6 bis 8 µ dick, beginnend mit der kleinen Magenkrümmung und parallel zu der langen Magenachse , hergestellt wurden. In den Magengrund- und Magenkörperschnittpräparaten mit einer Gesamtmasse 50-90 mg wurde der Gehalt an Eiweissstoff und Nucleinsauren ermittelt. Die Untersuchungsergebnisse sind in Tabellen 10 und 11 angeführt.

Die Tabellen 10 und 11 weisen einen offensichtlichen Vorteil der anzumeldenden Komposition im Vergleich zur Bernsteinsäure hinsichtlich der Stimulierung der funktionellen Aktivität der Magendrüsen und der Synthese von Makromolekülen in den Magen- und Lebergeweben von Mäusen aus. Somit ergibt sich die Stimulierung der Magensekretion und der fermentativen Aktivität der Succinatdehydrogenase in den Magen- und Lebergeweben durch die anzumendelde Komposition (s. Tabelle 7) aus der Aktivierung von nuklearen Prozessen darin.

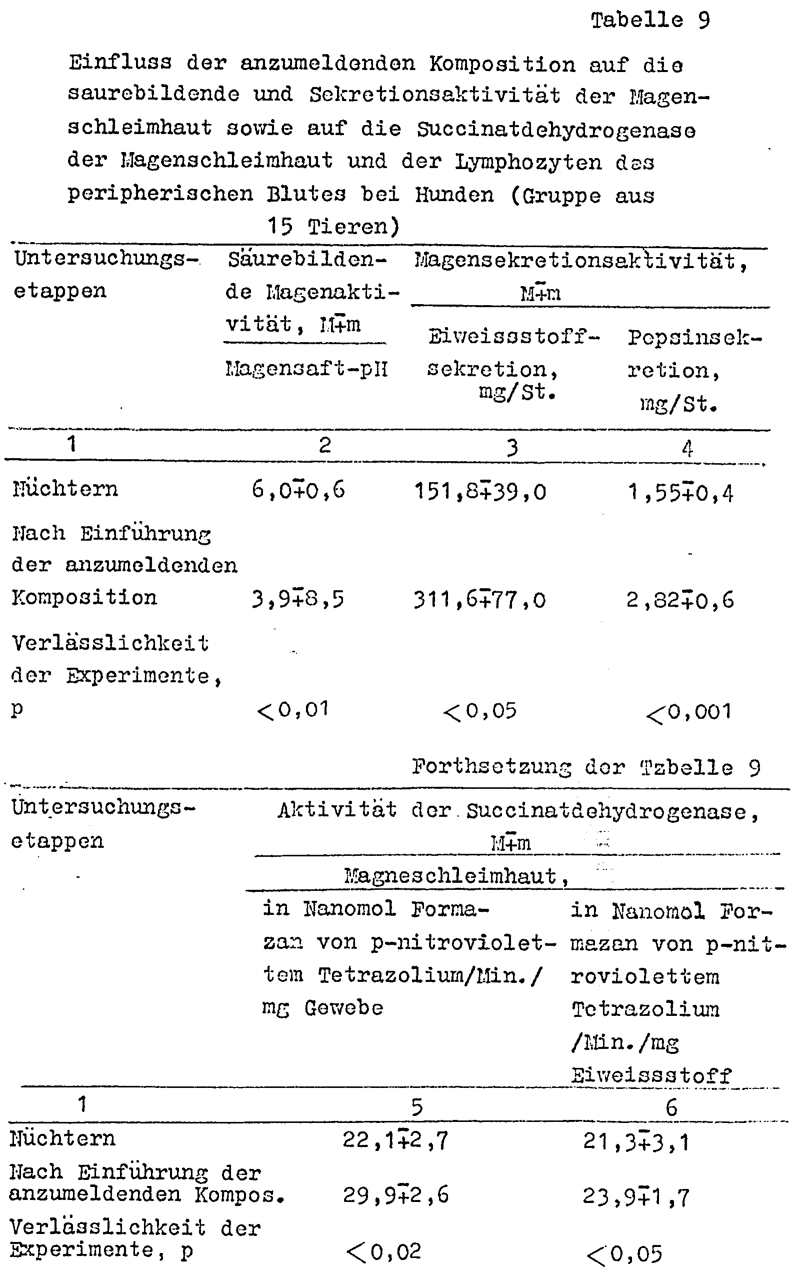

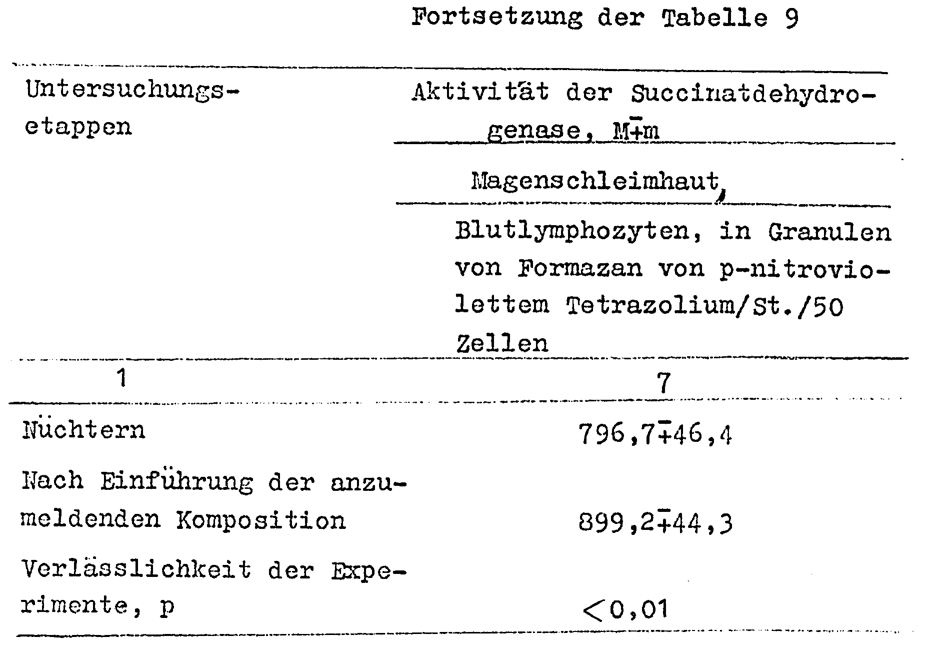

**Tabelle 10**

| Einfluss der anzumeldenden Komposition und der Bernsteinsäure auf die säurebildende und Sekretionsaktivität der Magenschleimhaut bei Mäusen | | | | |
|---|---|---|---|---|
| Gruppen von Tieren | Anzahl der Tiere in der Gruppe | Säurebildende Magenaktivität, M∓m | Sekretionsaktivität des Magens, M∓m | |
| | | pH-Wert des Mageninhaltes | Menge des Gesamteiweissstoffes im Mageninhalt, mg | Pepsinmenge im Mageninhalt, µg |
| Kontrolle | 25 | 5,5∓0,2 | 2,6∓0,4 | 14,4∓1,2 |
| Gruppe 1 (anzumeld. Komposit.) | 20 | 5,0∓0,3 | 4,8∓0,6 | 23,3∓2,0 |
| Gruppe 2 (Bernsteinsaure) | 11 | 5,7∓0,2 | 3,9∓0,9 | 18,7∓2,1 |
| Zuverlässisskeit der Experimente, p | | - | <0,01 | <0,01 |

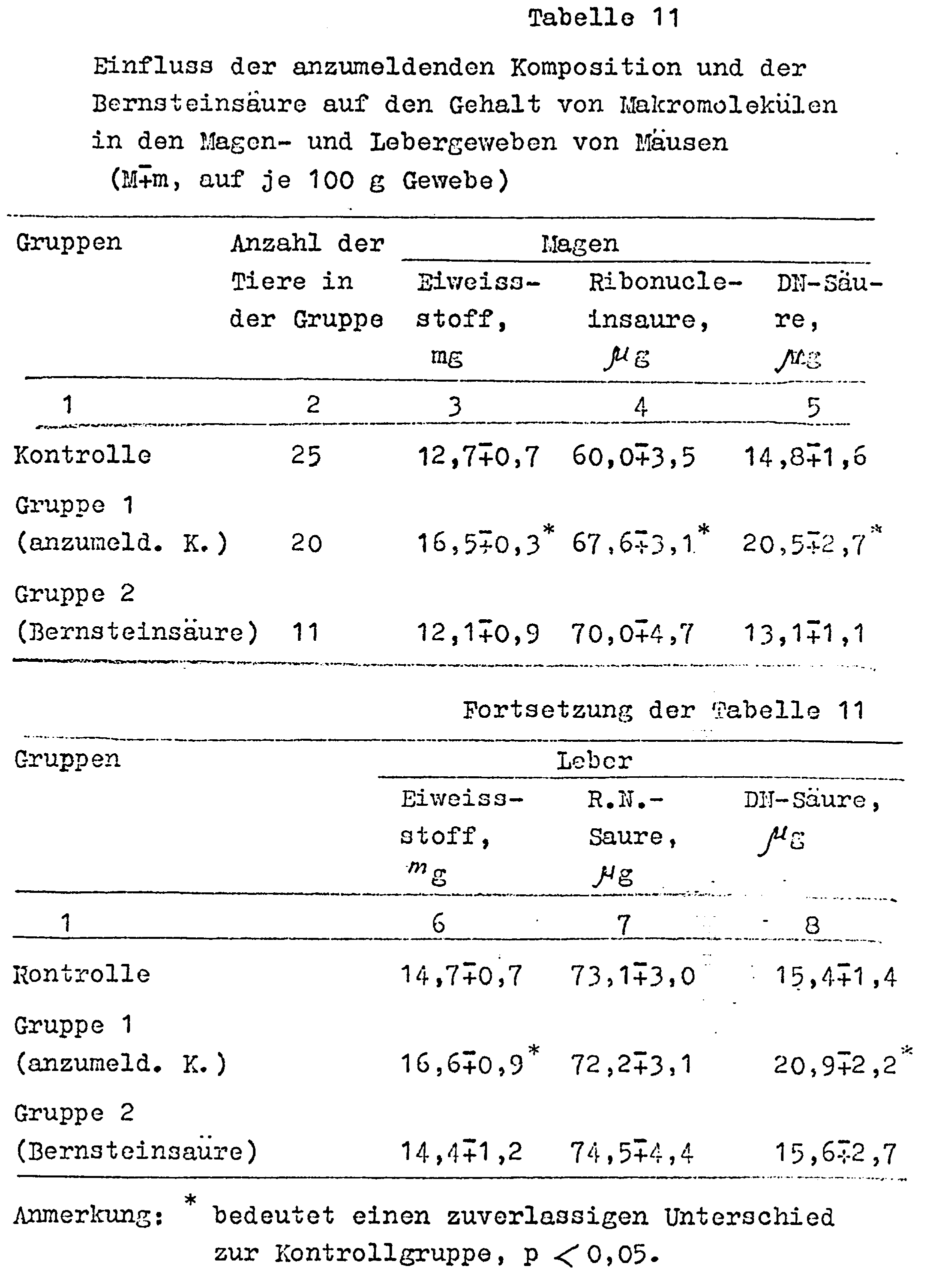

Eine Cholera-Prophylaxe-Wirkung der anzumeldenden Komposition wurde in vitro und in vivo unter Verwendung des Metschnikow-Vibrio untersucht. Eine Vibriokultur im Titer 10⁸ in einem Volumen von 0,1 ml wurde in 1 ml einer Lösung der anzumeldenden Komposition bei verschiedenen Konzentrationen deren Komponenten (s. Tabelle 12) eingebracht und bei 37^{o}C 1 Stunde lang inkubiert. Anschliessend nahm man eine Saat aus jedem Inkubationsmedium in Fläschchen mit einer alkalischen Pepton-Brühe vor, welche bei 37^{o}C thermostatiert wurden. Zur Identifizierung des Vibrios wurde nach 24 Stunden der Inhalt jedes Fläschechens auf ein alkalisches Nähragar ausgesät, das Agar wurde bei 37^{o}C thermostatiert. Nach 48 Stunden zählte man die Anzahl von Vibriokolonien auf dem Agar. Die Untersuchnungsergebnisse stehen in Tabelle 12. Sie zeigen, dass die anzumeldende Komposition bei den angegebenen Konzentrationen (welche denen in dem Magenbeutel bei peroraler Verabreichung der enzuneldenden Komposition ähnlich sind) das Wachstum und die Weiterentwicklung des Vibrios hemmt bzw. unterdrückt.

Die Experimente wurden an Mäusen mit Körpermasse 20-25 g durchgeführt. Der Versuchsgruppe (20 Tiere) wurde eine Lösung der anzumeldenden Komposition in einer Dosis von 3,75 mg/kg Körpermasse 3 mal täglich peroral eingeführt, wahrend eine Kontrollgruppe (20 Tiere) analogischerweise ein entsprechendes Wasservolumen bekam. Am zweiten Tag wurden der Versuchs- und der Kontrollgruppe 0,3 ml einer Suspension von Vibriokultur im Titer 10⁸ intragastral eingeführt. 20 Minuten zuvor bekam die Versuchsgruppe peroral eine Lösung der anzumeldenden Komposition in der obenangegebenen Dosis.

1 Stunde nach der Einführung der Vibriokultur wurden die Tiere dekaptiert, der Magen wurde freigelegt und isoliert, und man nahm die Aussaat des Mageninhalts in Fläschchen mit einer alkalischen Pepton-Brühe vor, die Fläschchen wurden bei 37^{o}C thermostatiert. Zur Identifizierung des Vibrios wurde der Inhalt nach 24 Stunden der Inhalt jedes Fläschchens auf ein alkalisches Nähagar ausgesät, das Agar wurde bei 37^{o}C thermostatiert. Nach 48 Stunden zählte man die Anzahl von Vibriokolonien auf dem Agar.

**Tabelle 12**

| Einwirkung verschiedener Konzentrationen der anzumeldenden Komposition auf die Lebensfähigkeit der Metschnikow-Vibrios | | | | |
|---|---|---|---|---|
| Komponenten der anzumeldenden Komposition | Konzentrationen der anzumeldenden Komposition, µ M | | | |
| Bernsteinsäure | 3 | 0,3 | 0,03 | 0,003 |
| Zitronensäure | 0,5 | 0,05 | 0,005 | 0,0005 |
| Anzahl der Vibriokolonien | 0 | 0 | 0 | 0 |

Als Ergibnis der Untersuchung wurde im Mageninhalt der Kontrolltiere eine Anwesenheit von Vibrios festgestellt, während bei den Versuchstieren kein Wachstum der Vibriokolonien zu verzeichnen war.

Eine akute Toxizität der anzumeldenden Komposition wurde an nicht reinrassigen weissen Mäusen mit Körpermasse 20-25 g und an nicht reinrassigen weissen Ratten, Körpermasse 200-250 g (Gruppen aus 30 Tieren) untersucht. Gefunden wurde, dass bei einer Einführung per os LD₅₀ der anzumeldenden Komposition für die Mäuse 5000 mg/kg Körpermasse und für die Ratten 4880 mg/kg Körpermasse und im Falle einer intraabdominalen Einführung für die Mäuse über 2400 mg/kg Körpermasse beträgt.

Eine chronische Toxizität der anzumeldenden Komposition wurde untersucht, indem sie Hunden mit Körpermasse 3-7 kg (eine Gruppe aus 8 Tieren) und nicht reinrassigen weissen Ratten mit Körpermasse 180-200 g (eine Gruppe aus 40 Tieren) in Dosen von 3, 7, 37 und 300 mg/kg Körpermasse per os 6 Monate lang eingeführt wurde. Die Untersuchung hat gezeigt, dass sie anzumeldende Komposition bei einer chronischen Einführung keine pathologischen Änderungen bezüglich des periphärischen Blutes, der Urinzusammensetzung, des morphologischen Bildes des Knochenmarks, der Innenorgane und des Gehirnes auslöst. Darüber hinaus zeigt die anzumeldende Komposition keineteratogene und mutagene Wirkung auf und beeinflusst nicht den Blutgerinnungsprozess, den Zustand des Immunsystems, die reproduktive Funktion und das Hypophyse-Adrenal-System.

Klinische Tests einer Antialkohol-Wirkung der anzumeldenden Komposition wurden in narkologischen stationären Heilungsstätten an 320 Alkoholikern und an 20 praktisch gesunden Menschen durchgeführt.

Erforscht wurde der Einfluss der anzumeldenden Komposition auf den Verlauf eines alkoholoschen Betrunkenheitszustandes bei 10 praktisch gesunden Menschen. Der Einfluss wurde anhand von Experteneinschätzungen und temporalen Sprechcharakteristika der zu untersuchenden Personen ausgewertet. Vor der Durchführung der Untersuchungen wurde der Gesundheitszustand der Patienten protokolarisch fixiert, darunter deren Schlafqualität, Ermüdungszustand, die Tätigkeit des Herz-Gefäss-Systems sowie der Zeitpunkt deren jüngsten Alkoholeinnahme. Die anzumeldende Komposition und Placebo wurdenden zu untersuchenden Personen in einer Dosis 3,75 mg/kg Körpermasse in Gelatinekapseln 20 Minuten vor der Einnahme einer 40% igen Alkohollösung in einer Dosis von 0,8 g/kg Körpermasse verordnet. Ein und derselbe Patient wurde in seinem Betrunkenheitszustand vor dem Placebo-Hintergrund sowie nach der Einnahme der anzumeldenden Komposition observiert. Nach der Alkoholeinnahme wurde die Entwicklungsdynamik der Betrunkenheit unenterbrochen kontrolliert, darunter deren Besonderheiten und Ausgeprägtheit. Durch Experteneinschätzungen wurde in 75% der Fälle die Wirkung der anzumeldenden Komposition im Vergleich mit Placebo genau ermittelt. Diese Wirkung kam besonders deutlich bei untersuchten Personen mit einer ausgepragten psychomotorischen Enthemmung zum Ausdruck. Bei Einnahme der anzumeldenden Komposition wurde eine Sprech- und Bewegungserregung praktisch nicht eingeschätzt, erhöhte sich die Selbstkontrolle in bezug auf Benehmen und Ausserungsart, schwand die Lassigkeit, Gehemmtheit und Disarthrie. Insgesamt kam die Wirkung der anzumeldenden Komposition bei ein und demselben Patienten im Vergleich zu Placebo in einem Ausgleich von Verhaltensreaktionen zum Ausdruck. In 50% der Fälle wurde ein schnellerer im Vergleich mit Placebo Austritt aus dem betrunkenen Zustand, unter Verminderung oder vollständiger Abwesenheit von Anzeichen einer toxischen Nachfolge von Äthanol, nämlich Kopfschmerzen, Übelkeit, Schwäche, Appetitabnahme, festgestellt. Darüber hinaus scrätzten die zu untersuchenden Personen bei Einnahme der anzumeldenden Komposition ihren Betrukenheitszustand subjektiv als ungewöhnlich ein, bei welchem die Selbstkontrolle in Benehmen und Äusserungsart erhalten geblieben war.

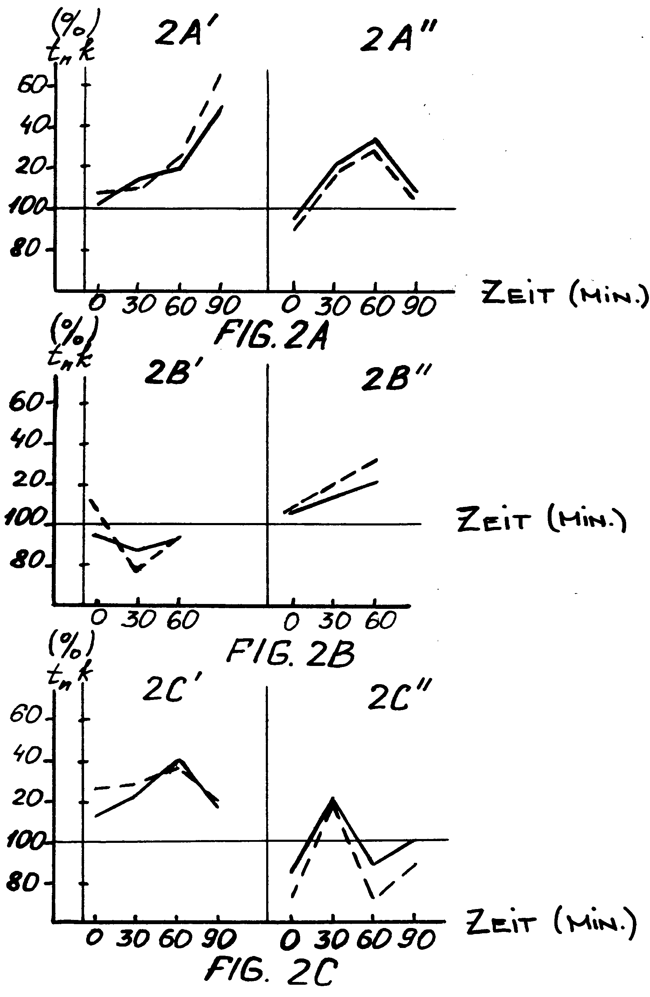

Eine Antialkoholwirkung der anzumeldenden Komposition bei denselben Untersuchungspersonen wurde anhand von temporalen Sprechcharaktoristiken eingeschätzt. Deren Analyse ermöglichte es, die Patienten in drei Versuchsgruppen einzuteilen. Die Untersuchungsergebnisse sind in Fig. 2A, 2B und 2C dargestellt, worin tₙ die Gesamtdauer von Sprechpausen, in % zum Hintergrund; k die relative Pausendauer, in % zum Hintergrund ; 100% den Hintergrund, d.h. das arithmetische Mittel der individuellen Sprechcharakteristika bei den Patienten im Ruhezustand in 3 Trainingstagen, bedeuten; die ausgezogene Linie ist Änderung der Gesamtdauer der Sprechüausen, die punktierte Linie bedeutet die relative Pausendauer bei den Versuchspersonen im Verlage des Experiments (p <0,05).

Dabei ist jede Fig. ist anschaulichkeitschalber in zwei Teile eingeteilt, wo 2A', 2B' und 2C' Änderungen der Parameter des Redeflusses bei den Patienten nach Einnahme von Alkohol mit Placebo angeben und 2A'', 2B'' un 2C'' dasselbe, aber nach Einnahme von Alkohol Mit der anzumeldenden Komposition veranschaulichen. Bei den Patienten der Gruppe 1 ändern sich die beiden Parameter nach der Alkohol+Placebo-Einnahme gleichgerichtet, sie tendieren nämlich recht deutlich zu einer Verlangsamung: Die Pausengesamtdauer nach 90 Minuten nach der Einnahme steigt um 53% und die relative Pausendauer wächst um 68% gegenüber dem Hintergrunf an (Fig. 2A, Teil 2A'). Nach der Alkoholeinnahme gemeinsam mit der anzumeldenden Komposition betragen die Änderungen dieser Parameter in der 60. Minute 31 bzw. 28% gegenüber dem Hintergrund, nach 90 Minuten gehen sie fast auf die Ausgangsgrösse zurück (Fig. 2A, Teil 2A'').

Bei den Patienten der Versuchsgruppe 2 gab es nach ihrer Alkoholeinnahme sowohl mit Placebo als auch mit der anzumeldenden Komposition geringe Parameterabweichungen des Sprechflusses gegenüber dem Hintergrund, ihre Tendenz aber war unterschiedlich (Fig. 2B, Teile 2B' und 2B''). Dieselbe deutliche Unterschiedlichkeit der Parameter ergaben die Untersuchungspersonen der Gruppe 3. Trotz des Unterschiedes der Ausgangsniveaus zeigt die Parameterdynamik in der 60. Minute eine recht deutliche Beschleunigung (Fig. 2C, Teil 2C') oder eine Verlangsamung (Fig. 2C, Teil 2C'') des Sprechflusses.

Es sei betont, dass die Patienten der Gruppen 1 und 3 im Falle der Alkoholeinnahme mit der anzumeldenden Komposition eine ausgeprägte Beschleunigung der Sprechnormalisierung aufzeigen. Die anzumeldende Komposition ergibt somit eine ausgeprägte alkoprotektorische detoxierende Wirkung.

Untersucht wurde ein Einfluss der anzumeldenden Komposition auf die Dynamik des Alkoholanteils in der Ausatmungsluft (unter Umrechnung in Alkoholgehalt im Blut der Patienten), und zwar bei 10 praktisch gesunden Menschen. Die Untersuchungspersonen bekamen eine wässrige Lösung der anzumeldenden Komposition in der Dosis 3,75 mg/kg Lörpergewicht 20 Minuten vor der Einnahme eines 40%igen Äthanols in einer Dosis von 0,5 g/kg Körpermasse. Der Alkoholgehalt in der Ausatmungsluft wurde nach bestimmten Zeitintervalen nach der Alkoholeinnahme gemessen. Die Untersuchungen zeigten, dass die antitoxische Wirkung der anzumeldenden Komposition durch eine beschleunigte Entfernung von Äthanol aus dem Ornismus und einen Anstieg der Konstante der Äthanolelimimie nierung von 0,1+0,25 auf 0,6+0,8 g· Stunde⁻¹ (p <0,05) bedingt ist.

Untersucht wurde eine Einwirkung der anzumeldenden Komposition auf den Verlauf des alkoholischen Abstinenzsyndroms bei Alkoholismuskranken in 2. und 3. Stadium, die ins Krankenhaus zwecks Kupierung ihrer periodoschen Trunksucht eingeliefert wurden. Sämtliche Patienten gingen ins Krankenhaus in einem Betrunkenheitszustand mit einem Äthanolgehalt im Blut 1,5-3,5 g/kg Körpergewicht ein. Die Krankengruppe 1 bekam eins standardmässige Therapiebehandlung, welche in Verabreichung von Neuroleptika, Tranquilisatoren und Antidepressanten bestand. Die Patientengruppe 2 bekam die Standardtherapiebehandlung gemeinsam mit der anzumeldenden Komposition in Form einer Lösung in einer Dosis 3-4 mg/kg Körpermasse auf einmal (Tagesgesamtdosis betrug 9-20 mg/kg Körpermasse), Gruppe 3 hatte lediglich die anzumeldende Komposition in den obigen Dosen einzunehmen.

Der Zustand der Patienten wurde anhand von summarischen Punkten, die den Ausprägegrad der wichtigsten klinischen Symptome eines alkoholischen Abstinenzsyndroms widerspiegeln, eingeschätzt. Der Ausprägegrad eines jeden Symptoms wurde nach einer 4-Punkt-Skala (0-3 Punkte) ausgewertet. Die erhaltenen Untersuchungsergebnisse sind in Tabelle 13 angeführt. Wie die Tabelle zeigt, steht die anzumeldende Komposition nach ihrer alkoholdetoxierenden Wirkung nicht hinter der recht starken Therapie, welche in manchen Fällen mehrere unerwünschte Nebennachwirkungen mit sich bringt (Enzephalopathie, Tremor u.a.m.).

**Tabelle 13**

| Einfluss der anzumeldenden Komposition auf den Zustand der Alkoholismuskranken bei Kupierung des alkoholischen Abstinenzsyndroms (in Punkten von 0 bis 3, M∓m) | | | | |
|---|---|---|---|---|
| Patientengruppen | Krankenanzahl in der Gruppe | Untersuchungsetappen | | |
| | | Vor Präparateinnahme | am 2.Tag der Präparateinnahme | am 3.Tag der Präparateinnahme |
| Gruppe 1 (Standardtherapie) | 25 | 12,2∓0,91 | 6,4∓0,95 | 2,1∓0,23 |
| Gruppe 2 (Standardtherapie + anzumeld. Komposition) | 27 | 13,5∓1,2 | 5,6∓0,65 | 2,3#-/+#0,55 |
| Gruppe 3 (anzumeld. Komposit.) | 11 | 14,4∓2,4 | 5,6∓1,00 | 1,7∓0,70 |

Es wurde gefunden, dass bei Patienten im Zustand der Betrunkenheit nach Einnahme der anzumeldenden Komposition die Erregung schwand, eine Selbsteinschätzung aufkam und die Patienten eine Erhellung im Kopfe'' deklarierten. Ausserdem waren am ersten Tag der Einnahme der anzumeldenden Komposition eine ausgeprägtere Verbesserung des Allgemeinbefindens und eine Appetitzunahme zu verzeichnen.

Eine Untersuchung der Geschwindigkeit der Rechenoperationen bei den Patienten (Addition von Paaren einstelliger Zahlen, insgesamt 184 Zahlenpaare) zeigte, dass nach 10 bis 18 Stunden nach der Einlieferung ins Krankenhaus sämtliche Patienten der Gruppe 3, die die anzumeldende Komposition bekommen hatten, imstande waren, eine Rechenaufgabe, zu bewältigen, und die Rechenoperationsgeschwindigkeit betrug 32∓1,2 Zeichen pro Minute. In Gruppe 1 betrug diese 19∓1,8 Zeichen (p 0,001), während 25% der Patienten die Aufgabe infolge einer eindeutig ausgeprägten Hemmung nicht erfüllen konnten. Die Unterschiede in der Geschwindigkeit der Aufgabeerfüllung wurden in den genannten Gruppen auch am 3. Tag der Behandlung nachgewiesen.

Eine Wirkung der anzumeldenden Komposition an Alkoholismuskranken im 2. und 3. Stadium, welche ambulatorisch behandelt wurden, wurde untersucht. Eine Gruppe 1 wurde während einer Periode eines täglichen Alkoholgebrauchs in einer Dosis von 1-1,5 g/kg Körpergewicht, während eine Gruppe 2 in einer Periode der Trunksucht beobachtet. Die anzumeldende Komposition wurde den Patienten in einer Dosis von 3-4 mg/kg Körpergewicht 3 bis 4 mal täglich 3-4 Tage lang während des Alkoholgebrauchs und 3 bis 4 Tage lang nach der Einstellung des Alkoholgebrauchs verordnet. Bei den Patienten der Gruppe 1 war bei der Einnahme der anzumeldenden Komposition eine Änderung des Krankheitsbildes des Betrunkenheitszustandes zu verzeichen: Das Eupheriestadium verlief nämlich ohre ausgeprägte Enthemmung, die Selbstkontrolle wurde besser, das aggressive Verhalten ging sichtlich zurück. Praktisch alle Patienten zeigten Appetitzunahme, ihre postintoxikationsbedingten Verstimmungen verminderten sich bedeutend, die früher zu einem Katzenjammertrieb angeregt hatten. Als Ergebnis erwies sich eine Verminderung des Alkoholgebrauchs, bis auf eine vollkommene Gebrauchseinstellung, sowie eine erhöhte Arbeitsfähigkeit. Festgestellt wurde ferner, dass falls die anzumeldende Komposition in der Remissionsperiode vor einem Alkoholgebrauch eingenommen wurde, eine einmalige Alkoholeinnahme, die bei solchen Kranken zu einem Rezidiv geführt hatte, ohne Nachfolgen verlief (das Abstinenzsyndrom fehlte) und zu keiner Krankheitsverschlimmerung führte (kei Rezidiv kam auf).

Die Krnaken der Gruppe 2 empfanden die Wirkung der anzumeldenden Komposition bereits nach 1-2 Stunden nach deren Einnahme, was in der Entstehung eines Zustandes der "Aufhellung" und "Ernüchterung" zum Ausdruck kam. In 75% der Fälle stellten die Patienten eine Verminderung und sogar ein Verschwinden des "Triebes" zum Alkohol fest und sagten dem Behandlungsarzt, dass man keine Lust zu trinken habe. Solch ein Zustand kam zu verschiedenen Fristen auf, nämlich 4 bis 12 Stunden nach der Einnahme der anzumeldenden Komposition, eine vollkommene Einstellung des Trunksuchtzustandes trat erst nach 1 bis 2 Tagen ein. Es wurde festgestellt, dass die Verwendung der anzumeldenden Komposition die Postintoxikationssymptomatik positiv beeinflusste. Es besserte sich der physische Zustand der Patienten, ihr Appetit nahm zu, es verminderte deren Schlaffheit, ein Gefühl der "Aufhellung im Kopfe" kam auf. In 15% der Fälle verband sich die Einnahme der anzumeldenden Komposition mit einem weiteren Alkoholgebrauch während 2 bis 3 Tage, die Tagesalkoholdosis ging allerdings wesentlich zurück.

Ein Einfluss der anzumeldenden Komposition auf Ambulanzpatienten im Zustand einer alkoholischen Betrunkenheit bei der Kupierung des alkoholischen Abstinenzsyndroms wurde untersucht. Die Krankengruppe 1 bekam eine Standardtherapie (Verabreichung von Tranquilisatoren, Antidepressanten und Natruimoxybutyrat). Gruppe 2 bekam die anzumeldende Komposition in Form von einer Lösung in einer Dosis von 3-4 mg/kg Körpergewicht auf einmal (Tugesgesamtdosis 9-20 mg/kg Körpergewicht). Wie auch bei der stationären Behandlung, wurde der Zustand der Patienten anhand einer Summe von Punkten ausgewertet, die den Ausprägungsgrad der wichtigsten klinischen Symptome das alkoholischen Abstinenzsyndroms widerspiegeln. Die erhaltenen Werte stehen in Tabelle 14.

**Tabelle 14**

| Einfluss der anzumeldenden Komposition auf den Zustand der Ambulanzpatienten bei der Kupierung des alkoholischen Abstinenzsyndroms (Punkte, M∓m) | | | |
|---|---|---|---|
| Krankengruppen | Krankenanzahl in der Gruppe | Untersuchungsetappen | |
| | | 1. Tag der Präparateinnahme | 2. Tag der Präparateinnahme |
| 1. Gruppe (Standardtherapie) | 10 | 9,9∓1,1 | 4,2∓1,2 |
| 2. Gruppe (anzumeldende Komposition) | 19 | 2,1∓0,09 | 0,8∓0,03 |
| Verlässlichkeit der Experimente, p | | <0,01 | <0,01 |

Wie aus Tabelle 14 ersichtlich, gab es bei der Patienten der Gruppe 2 bereits nach 12-18 Stunden (am 2. Behandlungstag) nach Einnahme der anzumeldenden Komposition praktisch keine Abstinenzerscheinungen. In 50% der Fälle konstatierten die Kranken bereits am 1. Behandlungstag das Verschwinden des "Triebs" zum Alkohol sowie Munterkeit und Appetitanregung.

Es wurde eine Einwirkung der anzumeldenden Komposition auf den Patientenzustand in der asthenischen Phase das alkoholischen Abstinenzsyndroms nach Verminderung akuter Krankheitserscheinungen untersucht. Die Kranken kamen ins stationäre Krankenhaus zur Unterbrechung des Trunksuchtszustandes oder bekamen eine ambulatorische Heilungsbehandlung. Die anzumeldende Komposition wurde ab 3. Tag nach der Einstellung des Alkoholgebrauchs vorordnet, in einer Dosis von 3-4 mg/kg Körpergewicht in Form einer Lösung 2-3 Male pro Tag 4 bis 6 Tage lang. Bei der Einnahme der anzumeldenden Komposition stellten die Patienten in 80-90% der Fälle eine deutliche Besserung ihres Allgemeinempfindens, eine Schlafnormalisierung, eine Munterkeit, Appetitnormalisierung, ein Schwinden dispeptischer Erscheinungen und einer Magenschwere fest. Es war eine Normalisierung des arteriellen Drucks in Fallen einer ausgepragten Hypotonie zu verzeichen.

Die durchgeführten Untersuchungen zeigten somit, dass die anzumeldende Komposition eine alkoprotektorische und eine alkoholdetoxierende Wirkung aufweist, die Alkoholentfernung aus dem Organismus beschleunigt, zur Alkoholgebrauchsverminderung anregt und die Intensivität eines pathologischen Triebs zum Alkohol senkt.

Klinische Tests der den Energiestoffwechsel stimulierenden Wirkung der anzumeldenden Komposition wurden an 20 Kranken mit Hypotonie-Erscheinungen wurden vorgenommen. Der systolische und der diastolische Druck bei den Kranken wurde vor und 20 Minuten nach der peroralen Einführung der anzumeldenden Komposition in einer Dosis von 3,75 mg/kg Körpergewicht gemessen. Die rhaltenen Werte sind in Tabelle 15 angegeben.

**Tabelle 15**

| Einfluss der anzumeldenden Komposition auf den arteriellen Druck bei Patienten mit Hypotonieerscheinungen | | |
|---|---|---|
| Untersuchungsetappen | Arterieller Druck, mm QS, M∓m | |
| | systolischer Druck | diastolischer Druck |
| Vor Einnahme der anzumeld. Komposition | 95∓2,9 | 62∓2,3 |
| Nach Einnahme der anzuzumeld. Komposition | 122∓3,3 | 78∓3,0 |
| Verlüssifkeit der Experimente, p | <0,05 | <0,05 |

Die Werte der Tabelle 15 zeigen, dass die anzumeldende Komposition den Energiestoffwechsel verstärkt, was in einem erhöhten Tonus der glatten Muskulatur der Gefässe zum Ausdruck kommt.

Klinische Test einer die säurebildenden und Sekretionsfunktion der Magenschleimhaut stimulierenden Wirkung der anzumeldenden Komposition wurden an 11 gesunden Menschen sowie an 498 Kranken mit verschiedenen gastroenterologischen Formen der Pathologie durchgeführt, und zwar mit chronischer Oberflächengastritis mit aufrechterhaltener Sekretion (97 Kranke); mit chronischer Oberflächengastritis mit verminderter Sekretion (74 Kranke); mit chronischer generalisierter atrophischer Gastritis mit verminderter Sekretion (177 Kranke); mit chronischer Gastritis mit erhöhter Sekretion vor Hintergrund einer Magengeschwurkrankheit (21 Kranke); mit chronischer Gastritis mit erhöhter Sekretion vor Hintergrund einer Zwölffingerdarmkrankheit (84 Kranke); mit diffuser gemischter Gastritis unter Schädigung des fundalen und des antralen Magenraums (30 Kranke) sowie mit chronischer hypertrophischer Gastritis (15).

Die Diagnose wurde aufgrund sämtlicher moderner Untersuchungsmethoden der gastroenterologischen Praxis verifiziert. Die anzumeldende Komposition wurde peroral in Form einer Lösung in 15 ml Wasser in einer Dosis von 4 mg/kg Körpergewicht eingeführt. Als Vergleichspräparate wurden Pentagastrin in einer Dosis von 6 µg/kg Körpergewicht und Hystamin in einer Dosis von 0,1 ml der 0,1%igen Lösung verwendet. Die Magensaft wurde in den beiden Sekrotionsphasen mit Hilfe einer ununter-brochenen-impulsiven Vakuumaspiration bei negativem Druck 50-60 mm QS nüchtern seine 20 Minuten nach Einfuhrung der anzumeldenden Komposition oder der Vergleichspräparate abgeführt. Zusätzlich nüchtern und nach Einführung der anzumeldenden Komposition oder der Vergleichspräparate wurde eine Innenmagen-pH-Metrie mit Hilfe eines Acidographen mit einer Zweioliven-Aspirationssonde vorgenommen. Vor und nach Einführung der anzumeldenden Komposition oder der Vergleichspräparate registrierte man das pH-Niveau, ermittelte die "alkalische Zeit" mittels Noller-Tests. Bei der Auswertung der pH-Gramme wurden das pH-Niveau, die "alkalische Zeit" (AZ), das Sekretionstempo der Wasserstoffionen (STWI) und die kinetische Säurebildungsfunktion (KSBF) bestimmt.

Ein Einfluss der anzumeldenden Komposition auf die Magensekretion bei Kranken mit chronischer Gastritis wurde untersucht. Die Untersuchungsergebnisse stehen in Tabelle 16. Sie zeigen, dass die anzumeldende Komposition ausgeprägt stimulierend auf sämtliche Anzeigewerte der Magensekretion einwirkt: es erhöhen sich das Sekretionsvolumen, die Säurezahl, der Anteil der saueren Komponente, die Fermentfreisetzung, der Säure- und Pepsinverbrauch, das pH-Niveau im Magen vermindert sich auf sauere Werte, es erhöht sich die KSBF, vermindert sich die AZ und verstärkt sich das STWI.

Eine Vergleichsuntersuchung der stimulierenden Wirkung von Pentagastrin und der anzumeldenden Komposition bei Kranken mit chronischer Gastritis wurde durchgeführt. Es wurde gefunden, dass der Einfluss der beiden Präparate auf sämtliche Anzeigewerte der Magensekretion (Magensaftvolumen, Säurezahl,partielle sauere Sekretion, Pepsingehalt, Säure- und Pepsinverbrauch) bei ein und denselben Patienten praktisch keine Unterschiede aufweist (p>0,5). Andererseits ergaben sich 12 vom den 50 Kranken mit chronischer Gastritis als refraktär zu der Wirkung von Pepsigastrin, d.h., nach dessen Einführung bleiben Säurezahl, Pepsingehalt, Salzsäure- und Pepsinverbrauch bei O-Werten. Bei einer intragastralen pH-Metrie bei diesen Kranken schwankte das pH-Ausgangsniveau im Magen zwischen 3,5 und 6,8 und erhöhte sich nach dem Noller-Test bis alkalischen Werten (8-9) und blieb so während der gesamten Untersuchungsperiode (1-1,5 Stunden). Nach Stimulierung der Magensekretion durch Pentagastrin änderte sich der pH-Wert im Magen dieser Kranken unwesentlich (von 6,51∓1,06 auf 4,69∓0,92; p >0,25) und der Noller-Test brachte keine Verminderung der AZ (p>0,5). Es wurde ein Einfluss der anzumeldenden Komposition auf die Magensekretion bei dieser Krankengruppe untersucht (Tabelle 17). Die Werte der Tabelle 17 zeigen, dass die Komposition, zum Unterschied von Pentagastrin, die Säure- und Fermentbildung stimuliert, die Kennwerte des Mageninhalts sich jedoch bei einer Sekretion durch das eine und das andere Präparat unwesentlich voneinander unter scheiden.

**Tabelle 16**

| Einfluss der anzumeldenden Komposition auf die Magensekretion bei Kranken mit chronischer Gastritis (Gruppe aus 50 Kranken) | | | |
|---|---|---|---|
| Kennwerte der Magensekretion | Basalsekretion | Stimulierte Sekretion | Zuverlässigkeit der Experimente,p |
| Mageninhaltsvolumen, 1/St., M∓m | 0,07∓0,005 | 0,11∓0,01 | <0,002 |
| Säurezahl, mMol/l, M∓m | 5,32∓1,37 | 46,90∓5,83 | <0,001 |
| Säure Sekretion, %, M∓m | 19,62∓0,95 | 38,84∓2,12 | <0,001 |
| Salzsäureverbrauch, mMol/St., M∓m | 0,28∓0,08 | 4,04∓0,56 | <0,001 |
| Pepsingehalt, mg/ml, M∓m | 0,55∓0,02 | 0,25∓0,03 | <0,001 |
| Pepsinverbrauch, mg/St., M∓m | 3,22∓1,28 | 19,85∓3,67 | <0,001 |
| pH, M∓m | 8,01∓0,18 | 2,04∓0,38 | <0,001 |
| KSBF, mg%, M∓m | 2,07∓0,37 | 9,93∓0,77 | <0,001 |
| AZ, Min., M∓m | 20,90∓2,52 | 12,36∓2,10 | <0,02 |
| STWI, in Einh. pH/Min., M∓m | 1,72∓0,36 | 3,73∓0,52 | <0,02 |

Solch ein Effekt der anzumeldenden Komposition hängt damit zusammen, dass sie die bioenergetischen und Wechselprozesse in der Magenschleimhaut aktiviert und die funktionelle Hemmung der Beleg- und Hauptzellen der Magendrüsen vermindert oder aufhebt und somit zu ihrer mehrmaligen Einführung zu Heilungszielen bei Kranken mit chronischer Gastritis bei einer pentagastrinrefraktären Achlorhydrie Verwendung finden kann.

**Tabelle 17**

| Einfluss der anzumeldenden Komposition auf die Magensekretion bei Kranken mit chronischer Gastritis bei einer pentagastrinrefraktären Achlorhydrie (Gruppe aus 12 Kranken) | | | |
|---|---|---|---|
| Kennwerte der Magen-sekretion | Durch Pentagastrin Stimulierte Sekretion | Durch die anzumeldende Komposit.stimulierte Sekretion | Zuverlässigkeit der Experimente, p |
| Mageninhaltsvolumen, 1/St., M∓m | 0,067∓0,15 | 0,111∓0,26 | 0,25 |
| Säurezahl, mMol/l, M∓m | 0∓0 | 32,0∓3,44 | 0,001 |
| Säure Sekretion, %, M∓m | 15,25∓0,86 | 25,25∓1,93 | 0,001 |
| Salzsäureverbrauch, mMol/St., M∓m | 0∓0 | 1,05∓0,19 | 0,001 |
| Pepsingehalt, mg/ml, M∓m | 0∓0 | 0,065∓0,02 | 0,01 |
| Pepsinverbrauch, mg/St., M∓m | 0∓0 | 1,21∓0,34 | 0,002 |

Wie klinische Untersuchungen zeigten, hängen die Effekte der anzumeldenden Komposition nicht so sehr von dem Pathologiecharakter als davon ab, in welcher Reihenfolge die genannten Präparate eingeführt wurden. Zum Beispiel unterscheidet sich der Einfluss der beiden Präparate auf die Kennwerte der Magensekretion bei Kranken mit chronischer Gastritis vor dem Hintergrund eine Magengeschwurs und eines Zwölffingerdarmgeschwürs praktisch nicht (p> 0,5). Eine Einführung von Pentagastrin einen Tag nach der anzumeldenden Komposition bringt jedoch eine verstärkte Wirkung auf die Kennwerte der Magensekretion bei den Kranken dieser Untersuchungsgruppe mit sich (Tabelle 18). Gleichzeitig unterscheidet sich die Wirkung der genannten Präparate im Falle der Einführung der anzumeldenden Komposition eine Woche nach Pentagastrin auf die Kennwerte der Magensekretion bei denselben Patienten praktisch nicht (p>0,5). Somit ruft die anzumeldende Komposition sogar bei ihrer einmaligen Einnahme eine Stimulierung der bioenergetischen Prozesse und der physiologischen Aktivität der Magenschleimhaut hervor, welche mindestens 24 Stunden weiterbesteht.

**Tabelle 18**

| Einfluss von Pentagastrin auf die Magensekretion bei dessen Einführung 24 Stunden nach der anzumeldenden Komposition bei Kranken mit chronischer Gastritis vor der Hintergrund eines Magengeschwürs und eines Zwölffingerdarmgeschwürs (Gruppe aus 24 Kranken) | | | | |
|---|---|---|---|---|
| Kennwerte der Magensekretion | Busalsekretion | Durch die anzumeld. Kemposit. stimulierte Sekretion | Durch Pentagastrin stimulierte Sekretion | Zuverlässigkeit der Experimente, p |
| Sekretionsvolumen, ml, M∓m | 56∓4,2 | 120∓6,3 | 178∓12,7 | 0,05 |
| pH, M∓m | 3,5∓0,29 | 1,7∓0,11 | 1,4∓0,14 | 0,05 |
| Salzsäureverbrauch, mMol/St., M∓m | 2,0∓0,27 | 9,2∓0,68 | 17∓1,44 | 0,05 |
| Pepsinverbrauch, mg/St., M∓m | 4,5∓0,6 | 5,9∓0,75 | 5,0∓1,05 | - |

Ein Einfluss der anzumeldenden Komposition auf die säurebildende Funktion der Magenschleimhaut bei Kranken mit verschiedenen Formen chronischer Gastritis (Tabelle 19) und bei einer Fraktionsanalyse des Mageninhalts bei praktisch gesunden Menschen (Tabelle 20) wurde erforscht, ferner bei Kranken mit chronischer Oberflächengastritis mit aufrecherhaltener Sekretion und mit chronischer atrophischer Gastritis mit verminderter Sekretion (Tabelle 21), bei Kranken mit chronischer Gastritis vor dem Hintergrund eines Magengeschwürs und Zwölffingerdarmgeschwürs (Tabelle 22).

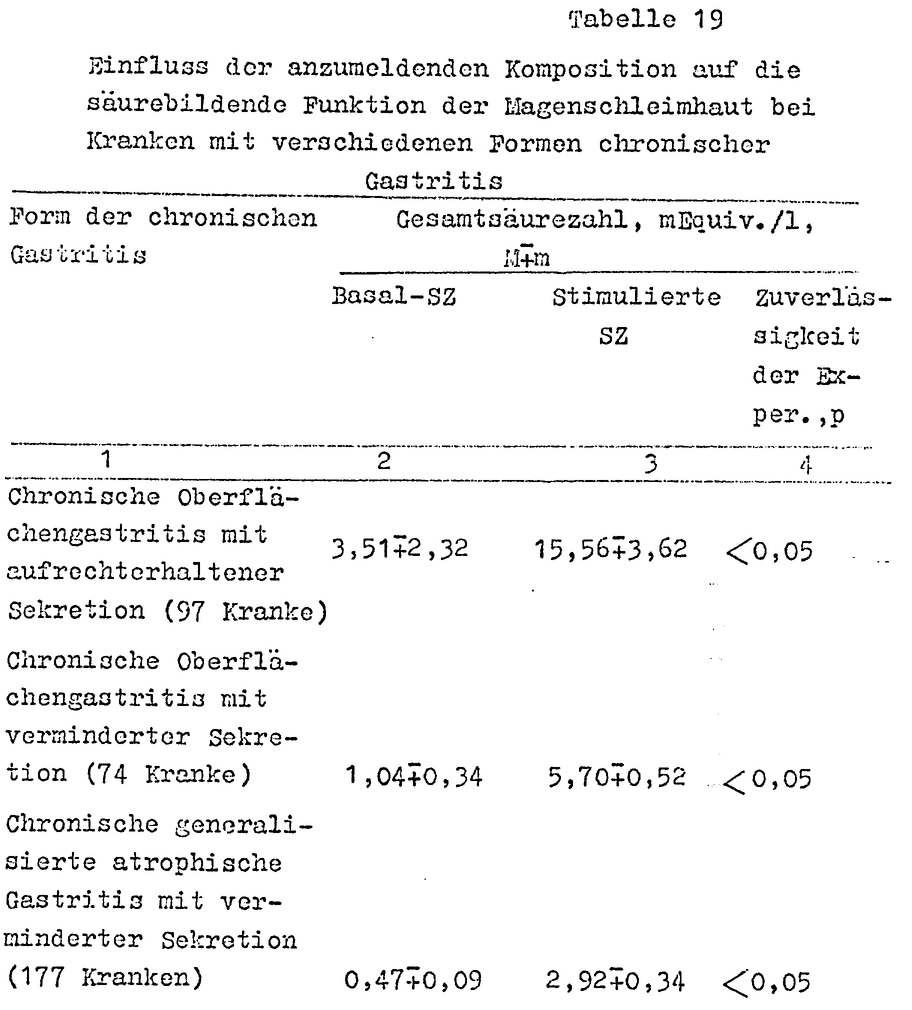

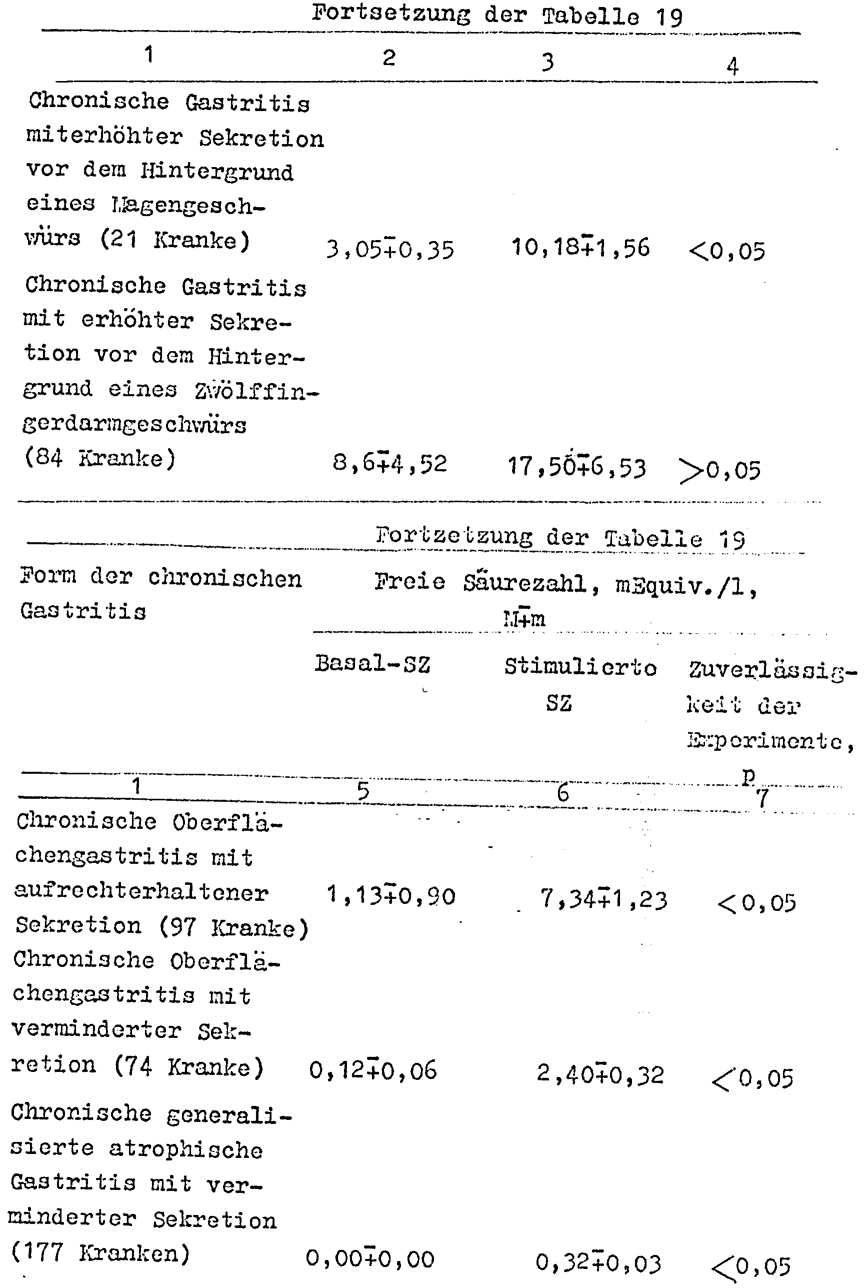

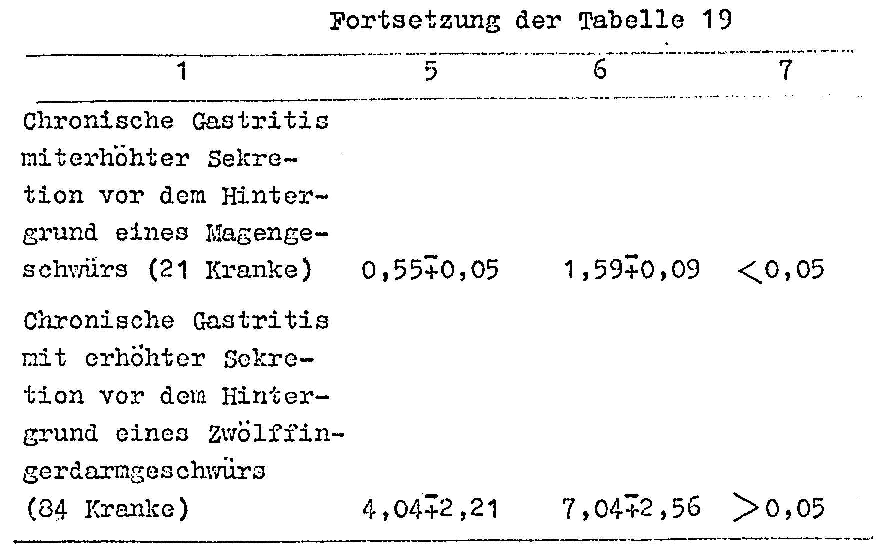

**Tabelle 20**

| Einfluss der anzumeldenden Komposition auf die säurebildende Funktion der Magenschliemhaut bei praktisch gesunden Menschen (Gruppe aus 11 Versuchspersonen) | | | |
|---|---|---|---|
| Nr. | der Magensaftportion | Freie Salzsäure, mEquiv./l, M∓m | Allgemeine Salzsäure, mEquiv./l, M∓m |
| 1. | Portion (0 Min.,Basalsekr.) | 6,6∓3,4 | 23,3∓16,3 |
| 2. | Portion (nach 15 Min.) | 7,0∓2,3 | 25,3∓9,0 |
| 3. | Portion (nach 30 Min.) | 11,3∓4,0 | 25,6∓13,3 |
| 4. | Portion (nach 45 Min.) | 11,3∓4,0 | 27,6∓13,3 |
| 5. | Portion (nach 60 Min.) | 5,6+2,3 | 18,6∓5,3 |
| Stimulierung durch die anzumeldende Komposition: | | | |
| 6. | Portion (nach 75 Min., stimulierte Sekretion) | 22,3∓7,0 | 89,3∓7,0 |
| 7. | Portion (nach 90 Min.) | 55,0∓24,3 | 81,3∓23,3 |
| 8. | Portion (nach 105 Min.) | 44,0∓6,6 | 72,3∓17,6 |
| 9. | Portion (nach 120 Min.) | 47,3∓11,0 | 64,6∓26,0 |

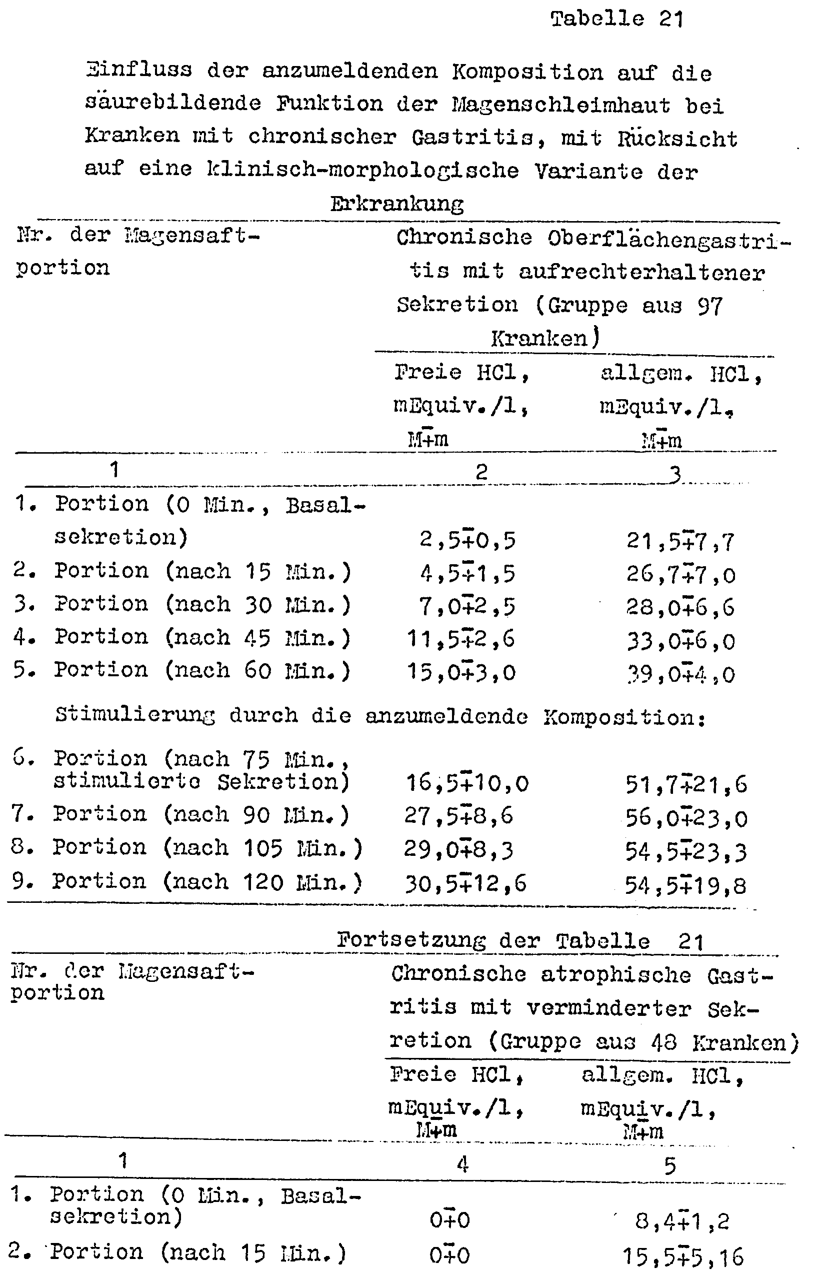

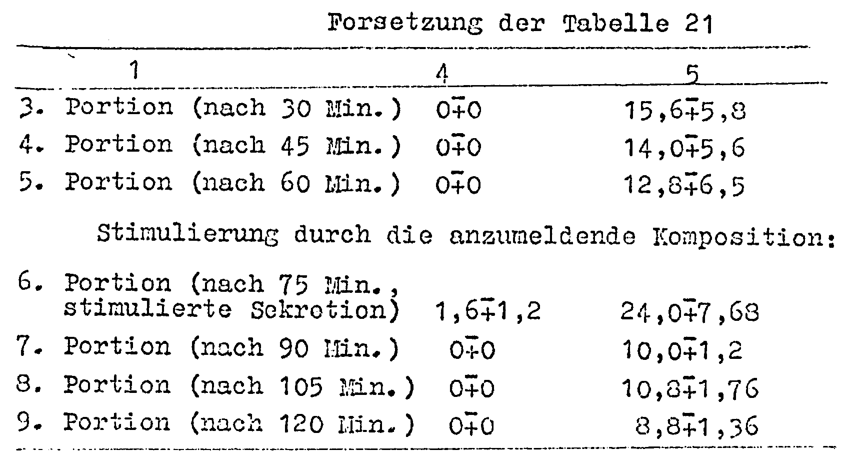

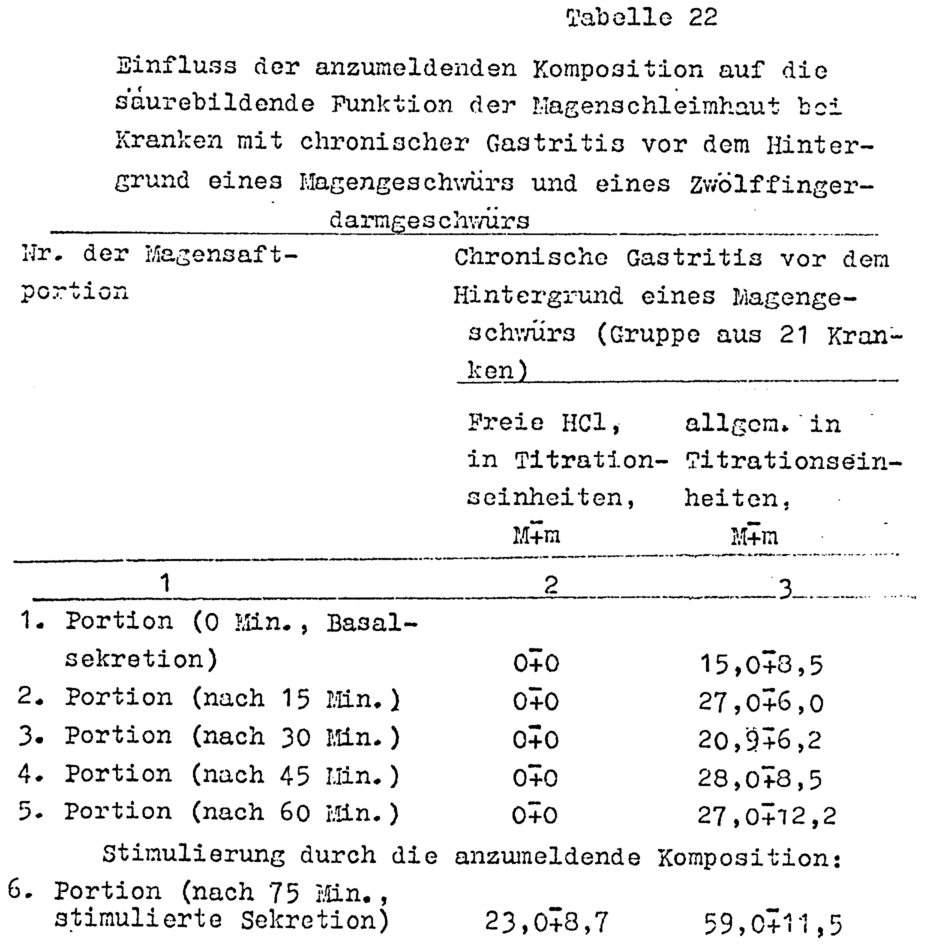

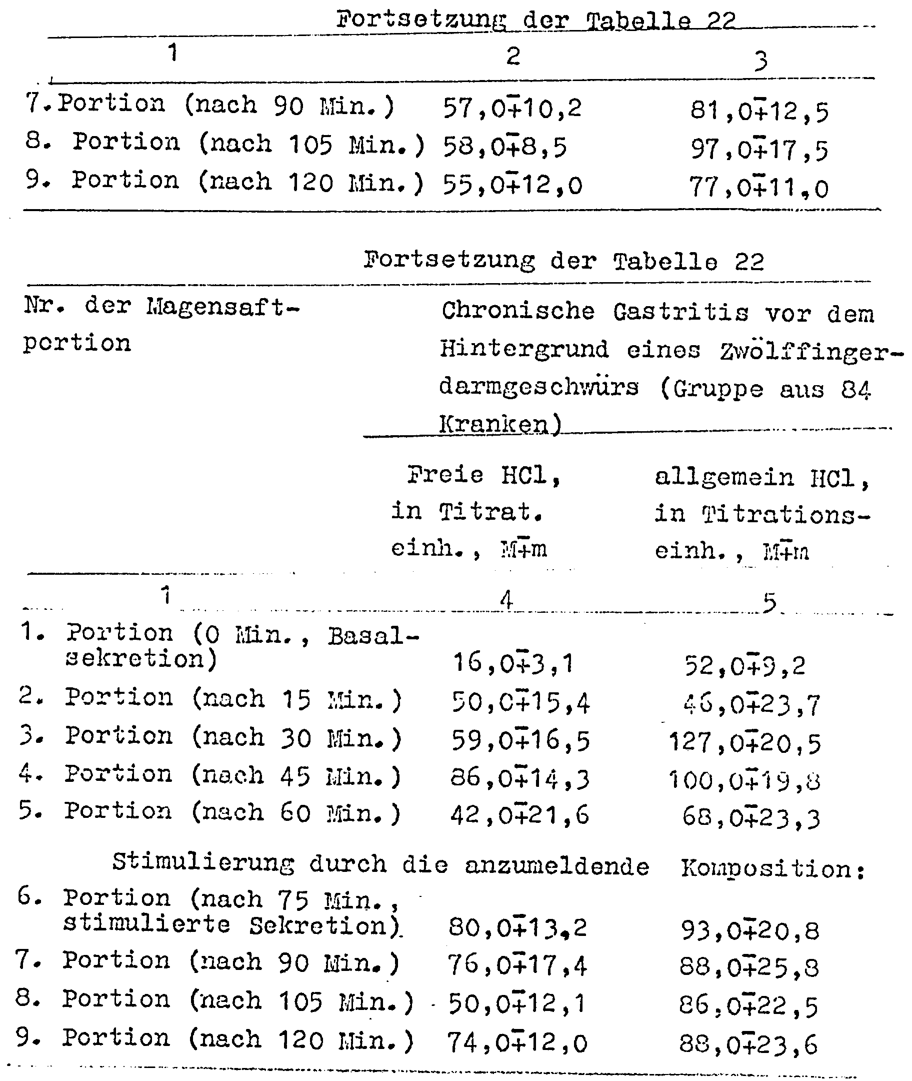

Die Werte der Tabellen 19, 20, 21 und 22 zeigen, dass die anzumeldende Komposition wirkt ausgeprägt stimulierend auf die säurebildende Funktion der Magenschleimhaut sowohl bei praktisch gesunden Menschen als auch bei Kranken mit verschiedenen Formen der chronischen Gastritis.

Es wurde eine Vergleichsuntersuchung der Einwirkung der anzumeldenden Komposition und von Hystamin auf die säurebildende Funktion der Magenschleimhaut bei praktisch gesunden Menschen durchgeführt. Zu diesem Zweck wurden deren Kennwerte der säurebildenden Funktion des Magens bei der Einführung der genannten Präparate in verschiedenen Reihenfolgen gemessen. Die Untersuchungsergebnisse stehen in Tabellen 23 und 24. Sie zeigen, dass die anzumeldende Komposition sowohl zeitlich als auch intensitätsmässig die Säuerbildung in der Magenschleimhaut genauso beeinflusst wie Hystamin. Die Komposition zeichnet sich jedoch vorteilhaft von Hystamin durch die perorale Einführung den Patienten sowie durch keine unerwünschten Nebenwirkungen aus.

Ein Einfluss der anzumeldenden Komposition auf die säurebildende Funktion der Magenschleimhaut bei chronischen Alkoholismuskranken wurde untersucht. Die in stationaren Verhältnissen durchgeführte Fraktionsuntersuchung des Mageninhaltes zeigte, dass am dritten Tag des alkoholischen Abstinenzsyndrom die säurebildende und Sekretionsfunktion des Magens (das Magensaftvolumen ist in den ersten Portionen ist auf 2-5 ml vermindert) bei den Kranken rapide zurückgeht und eine grosse Menge von Mucin vorhanden ist. Eine Einführung der anzumeldenden Komposition stimulierte bei den Patienten die säurebildende Funktion der Magenschleimhaut, eine Vergrösserung der Mageninhaltsmenge sowie die Mucinsekretion. Die Untersuchungsergibnisse sind in Tabelle 25 angeführt.

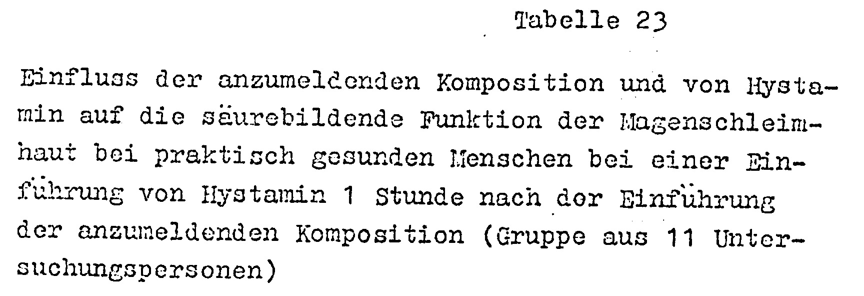

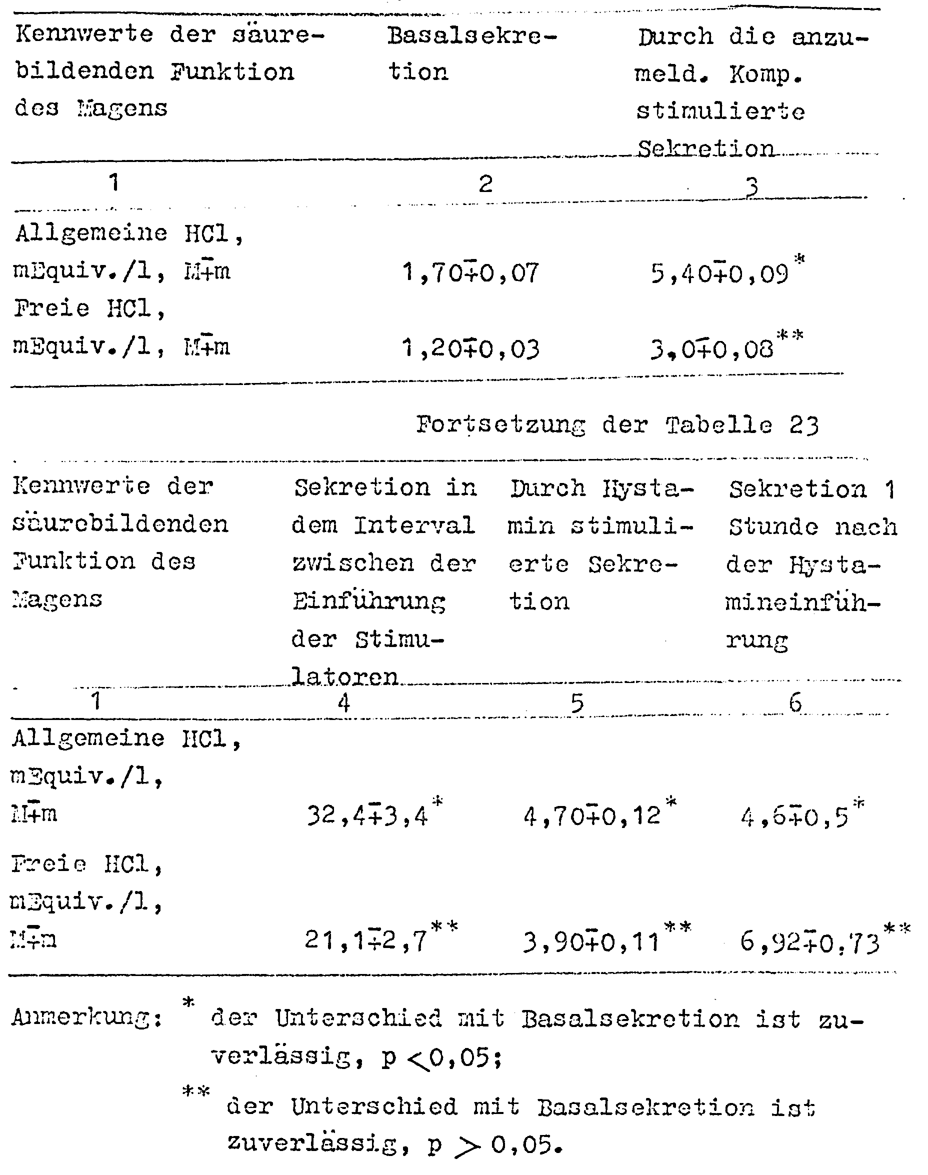

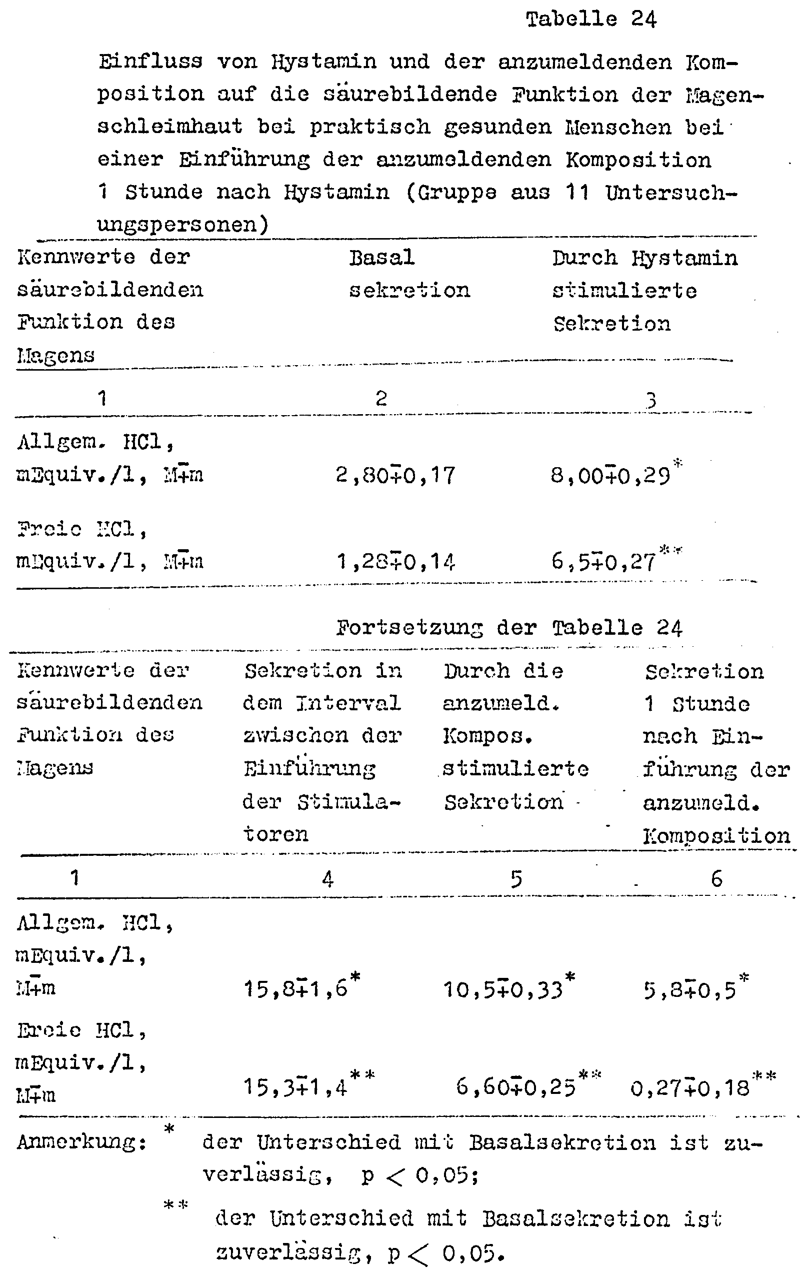

Als dieselbe Krankengruppe die anzumeldende Komposition als Heilmittel (in einer Tagesgesamtdosis von 8-12 mg/kg Körpergewicht) waren am Ende des ersten/Anfang des zweiten Untersuchungstages Appetitzunahme Schwindung eines Bitterkeitsbeigeschmacks, Normalisierung des Stuhlganges (kein Stuhlverstopfung mehr) zu verzeichnen, und eine 4- bis 6tägige Einnahme der anzumeldenden Komposition ermöglichte es, die säurebildende Funktion der Magenschleimhaut bei den Patienten zu normalisieren (Tabelle 26).

**Tabelle 25**

| Einfluss der anzumeldenden Komposition auf die säurebildende Funktion der Magenschleimhaut bei Kranken mit chronischem Alkoholismus am 3. Tag des alkoholischen Abstinenzsyndroms (Gruppe aus 10 Kranken) | | | |
|---|---|---|---|
| Nr. | der Magensaftportion | Freie HCl, mEquiv./l, M∓m | Allgem. HCl, mEquiv./l, M∓m |
| | 1 | 2 | 3 |
| 1. | Portion (0 Min., Basalsekretion) | 0,85∓0,39 | 8,37∓1,70 |
| 2. | Portion (nach 15 Min.) | 1,15∓0,39 | 11,60∓1,80 |
| 3. | Portion (nach 30 Min.) | 1,45∓0,38 | 11,50∓1,90 |
| 4. | Portion (nach 45 Min.) | 2,35∓0,53 | 14,20∓3,00 |
| 5. | Portion (nach 60 Min.) | 1,40∓0,33 | 11,60∓1,90 |
| Stimulierung durch die anzumeldende Komposition: | | | |
| 6. | Portion (nach 75 Min., stimulierte Sekretion) | 4,70∓1,30 | 19,30∓3,30 |
| 7. | Portion (nach 90 Min.) | 6,80∓1,20 | 21,30∓2,60 |
| 8. | Portion (nach 105 Min.) | 4,59∓1,10 | 21,30∓2,60 |
| 9. | Portion (nach 120 Min.) | 1,40∓0,60 | 10,20∓1,90 |

**Tabelle 26**

| Einfluss der anzumeldenden Komposition auf die säurebildende Funktion der Magenschleimhaut bei Kranken mit chronischem Alkoholismus nach 4- bis 6tägiger Einnahme der Komposition in Stadium des alkoholischen Abstinenzsyndroms (Gruppe aus 10 Kranken) | | | |
|---|---|---|---|
| Nr. | der Magensaftportion | Freie HCl, mEquiv./l, M∓m | Allgemeine HCl, mEquiv./l, M∓m |
| 1. | Portion (0 Min., Basalsekretion) | 5,96∓0,90 | 22,50∓1,30 |
| 2. | Portion (nach 15 Min.) | 6,08∓0,90 | 23,10∓2,20 |
| 3. | Portion (nach 30 Min.) | 9,62∓1,10 | 24,10∓1,70 |
| 4. | Portion (nach 45 Min.) | 11,22∓1,20 | 27,40∓1,70 |
| 5. | Portion (nach 60 Min.) | 13,05∓0,93 | 31,70∓1,68 |
| Stimulierung durch die anzumeld. Komposition: | | | |
| 6. | Portion (nach 75 Min., stimulierte Sekretion) | 32,50∓3,00 | 59,90∓4,30 |
| 7. | Portion (nach 90 Min.) | 58,80∓5,20 | 89,90∓7,10 |
| 8. | Portion (nach 105 Min.) | 52,60∓5,00 | 83,30∓5,60 |
| 9. | Portion (nach 120 Min,) | 46,10+5,00 | 76,50+5,00 |

**Tabelle 27**

| Einfluss der anzumeldenden Komposition auf die säurebildende Funktion der Magenschleimhaut bei Kranken mit chronischem Alkoholismus (Ambulanzbehandlung, Gruppe aus 20 Krnaken) | | | |
|---|---|---|---|
| Nr | der Magensaftportion | Freie HCl, mEquiv./l, M∓m | Allgemeine HCl, mEquiv./l, M∓m |
| | 1 | 2 | 3 |
| 1. | Portion (0 Min., Basalsekretion) | 3,52∓0,33 | 19,90∓0,89 |
| 2. | Portion (nach 15 Min.) | 4,02∓0,38 | 21,40∓0,78 |
| 3. | Portion (nach 30 Min.) | 4,71∓0,31 | 23,80∓0,66 |
| 4. | Portion (nach 45 Min.) | 4,94∓0,41 | 25,05∓0,53 |
| 5. | Portion (nach 60 Min.) | 4,52∓0,37 | 22,45∓0,42 |
| Stimulierung durch die anzumeld. Komposition: | | | |
| 6. | Portion (nach 75 Min., stimulierte Sekretion) | 8,95∓0,67 | 27,75∓0,48 |
| 7. | Portion (nach 90 Min.) | 10,70∓0,42 | 31,60∓0,60 |
| 8. | Portion (nach 105 Min.) | 11,70∓0,36, | 33,35∓0,36 |
| 9. | Portion (nach 120 Min.) | 12,30∓0,42 | 33,75∓0,78 |

**Tabelle 28**

| Einfluss der anzumeldenden Komposition auf die säurebildende Funktion der Magenschleimhaut nach 5- bis 7tägiger Einnahme bei Ambulanzkranken mit chronischem Alkoholismus (Gruppe aus 20 Kranken) | | | |
|---|---|---|---|
| Nr. | der Magensaftportion | Freie HCl, mEquiv./l, M∓m | Allgemeine HCl, mEquiv./l, M∓m |
| | 1 | 2 | 3 |
| 1. | Portion (0 Min., Basalsekretion) | 6,40∓0,57 | 22,65∓0,70 |
| 2. | Portion (nach 15 Min.) | 6,99∓0,60 | 24,00∓0,96 |
| 3. | Portion (nach 30 Min.) | 10,44∓0,60 | 27,60∓1,10 |
| 4. | Portion (nach 45 Min.) | 9,00∓0,55 | 28,15∓1,68 |
| 5. | Portion (nach 60 Min.) | 5,90∓0,35 | 19,25∓1,02 |
| Stimulierung durch die anzumeldender Komposition: | | | |
| 6. | Portion (nach 75 Min., stimulierte Sekretion) | 21,35∓0,72 | 94,65∓2,10 |
| 7. | Portion (nach 90 Min.) | 53,85∓3,50 | 89,10∓5,90 |
| 8. | Portion (nach 105 Min.) | 43,05∓1,56 | 72,15∓3,00 |
| 9. | Portion (nach 120 Min.) | 44,60∓2,02 | 77,20∓4,20 |

Es betont, dass bei den Ambulanzkranken, welche die anzumeldende Komposition nicht einnahmen, zu diesen Fristen keine spontane Wiederherstellung der säurebildenden Funktion der Magenschleimhaut zu verzeichnen war.

### Industrielle Verwendbarkeit

Die anzumeldende Komposition weist eine antialkoholische, den Energiestoffwechsel, die säurebildende und Sekretionsfunktion der Magenschleimhaut stimulierende, strahlungsprotektorische und Anticholera-Wirkung auf und findet in der Medizin als Arzneipräparat zur Heilung des Alkoholismus, eine akuten alkoholischen Betrunkenheitszustandes und dessen Nachwirkungen, als Diagnostikum zur Ermittlung der säurebildenden und Sekretionsfunktion der Magens, zur Heilung der Hypotomie, anacider und hypoacider Gastritis, asthenischer Zustände, inbesondere im Falle des Alkoholismus und grosser körperlicher Belastungen (in Sportmedizin, allerlei Produktionstätigkeit im Betrieb), ferner als Strahlungsprotektor zum Schutz vor Strahlungsbeeinflussungen sowie zur Prophylaxe von Cholera Verwendung.

## Claims

1. Pharmazeutische Komposition mit antialkoholischer, den Energiestoffwechsel, die säurebildende und Sekretionsfunktion der Magenschleimhaut stimulierender, strahlungsschützender und Anticholera-Wirkung, bestimmt für perorale Einführung, **dadurch gekennzeichnet,** dass sie als Wirkstoff eine Mischung von Bernstein- und Zitronensäure oder deren pharmazeutisch verträglichen Salzen enthält.

2. Pharmazeutische Komposition nach Anspruch 1, in Form von Lösung oder Pulver, **dadurch gekennzeichnet,** dass sie von 0,1 bis 0,3 g Bernsteinsäure und von 0,025 bis 0,085 g Zitronensäure oder deren pharmazeutisch verträgliche Salze für 1 Dosis enthält.

3. Pharmazeutische Komposition nach Ansprüchen 1 und 2, **dadurch gekennzeichnet,** dass sie als pharmazeutisches Lösungsmittel Wasser oder alkalisches Wasser enthält.

4. Verfahren zur Prophylaxe und Heilung eines alkoholischen Betrunkenheitszustandes und eines alkoholischen Abstenzsyndroms durch perorale Einführung einer effektiven Menge der pharmazeutischen Komposition nach Anspruch 1.

5. Verfahren zur Stimulierung des Energiestoffwechsels durch perorale Einführung einer effektiven Menge der pharmazeutischen Komposition nach Anspruch 1.

6. Verfahren zur Stimulierung und Diagnostik der säurebildenden und Sekretionsfunktion der Magenschleimhaut durch perorale Einfuhrung einer effektiven Menge der pharmazeutischen Komposition nach Anspruch 1.

7. Verfahren zum Schutz vor Strahlungsbeschädigungen durch perorale Einfuhrung einer effektiven Menge der pharmazeutischen Komposition nach Anspruch 1.

8. Verfahren zur Prophylaxe von Cholera durch perorale Einführung einer effektiven Menge der pharmazeutischen Komposition nach Anspruch 1.
